# EUROPEAN PATENT APPLICATION

(11) **EP 4 434 513 A1**
(43) Date of publication of application: **25.09.2024**
(21) Application number: 22894765.1
(22) Date of filing: 15.11.2022
(51) Int. Cl.: A61K 9/00, A61K 9/20, A61K 9/48, A61K 31/506, A61P 31/14, A61P 37/06, A61P 3/00

(54) **NANOCRYSTALLINE PREPARATION OF ROCK2 INHIBITOR AND PREPARATION METHOD THEREFOR**

(30) Priority: 16.11.2021 CN 202111358608; 03.11.2022 CN 202211429651
(71) Applicant: Beijing Tide Pharmaceutical Co., Ltd., Beijing 100176 (CN)
(72) Inventor: LU, Di, Beijing 100176 (CN); ZHU, Zhaolu, Beijing 100176 (CN); ZHANG, Zhibing, Beijing 100176 (CN); NIU, Shengpan, Beijing 100176 (CN); LU, Yongjie, Beijing 100176 (CN); XU, Jiajia, Beijing 100176 (CN); ZHANG, Shasha, Beijing 100176 (CN)
(74) Representative: Novitas Patent AB
(86) International application number: PCT/CN2022/131872
(87) International publication number: WO 2023/088231

(57) **Abstract**

A nanocrystalline preparation and a preparation method therefor, the nanocrystalline preparation comprising a ROCK2 inhibitor and a stabilizer. The present invention also relates to use of the nanocrystalline preparation in the prevention, alleviation, and/or treatment of selected diseases and medical conditions, especially diseases such as idiopathic pulmonary fibrosis, fatty liver disease and/or steatohepatitis, post-hematopoietic stem cell transplantation graft versus host disease or viral infection.

## Description

### TECHNICAL FIELD

The present disclosure relates to the field of medicine. Specifically, the present disclosure relates to a nanocrystal formulation of a ROCK2 inhibitor and a preparation method thereof.

### BACKGROUND

Idiopathic pulmonary fibrosis (IPF) is a progressive respiratory disease with pulmonary tissue fibrosis and reduction and loss of lung function as main clinical features. The median survival period is 2.5-3 years. For a ROCK2 target drug, the United States is conducting a phase II clinical study on IPF (Kadmon Holdings, Inc.), and the preliminary results confirm the safety and effectiveness of a ROCK2 inhibitor in the treatment of IPF.

[6-[4-[[4-(1H-pyrazol-4-yl)phenyl]amino]pyrimidin-2-yl]-1-methyl-1H-indole-2-yl]( 3,3-difluoroazetidin-1-yl)methanone is a new type of highly selective ROCK2 inhibitor with a new target and a new structural type completely independently developed by Beijing Tide Pharmaceutical Co., Ltd. From the perspective of patient compliance, an oral formulation is selected for the treatment of IPF. Its high selectivity for a target greatly reduces safety risks. Our company has obtained the patent for this compound in the United States, and has applied for compound patents in China, the European Union, Japan, South Korea, India, Canada, Australia and other countries and regions.

[6-[4-[[4-(1H-pyrazol-4-yl)phenyl]amino]pyrimidin-2-yl]-1-methyl-1H-indole-2-yl]( 3,3-difluoroazetidin-1-yl)methanone is a pale yellow to yellow solid powder with poor solubility and is insoluble in water and a buffer salt solution with pH 1.0~pH 6.8. It has poor physical properties and is prone to stickiness, static electricity and aggregation. Therefore, how to prepare a formulation of the compound and improve the dissolution of the product has become an urgent technical problem to be solved by those skilled in the art.

### SUMMARY

An object of the present disclosure is to provide a nanocrystal formulation of a ROCK2 inhibitor and a preparation method thereof, so as to improve the dissolution of the ROCK2 inhibitor. The specific technical solutions are as follows:

The present disclosure firstly provides a nanocrystal formulation, which comprises a ROCK2 inhibitor and a stabilizer, wherein the ROCK2 inhibitor is a compound of formula (I), 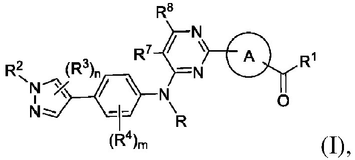 wherein
Ring A is 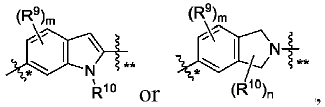 wherein the above group is connected to the pyrimidine ring through one of the two positions marked by * or **, and the other position is connected to the carbonyl group;
R⁹ and R¹⁰ at each occurrence are each independently selected from H, halogen, C₁₋₆ alkyl, C₂₋₆ alkenyl, C₃₋₁₀ carbocyclyl, 3-10 membered heterocyclyl, C₆₋₁₀ aryl, 5-14 membered heteroaryl, C₆₋₁₂ aralkyl, -C(=O)R⁵ and -C₁₋₆ alkylene-O(P=O)(OH)₂;
m at each occurrence is independently an integer of 0, 1, 2, or 3; and
n at each occurrence is independently an integer of 0, 1 or 2;
alternatively, ring A is 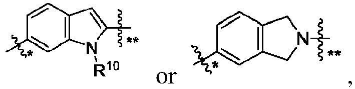 wherein the above group is connected to the pyrimidine ring through the position marked by *, and is connected to the carbonyl group through the position marked by **, wherein R¹⁰ is selected from H and C₁₋₆ alkyl, alternatively H or methyl;
R is selected from H and C₁₋₆ alkyl;
R¹ is 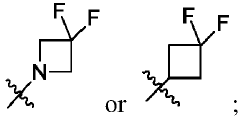
R² is selected from H and C₁₋₆ alkyl;
R³, R⁴, R⁷ and R⁸ at each occurrence are each independently selected from H, halogen, -NR⁵R⁶, -OH, C₁₋₆ alkyl and -OR⁵;
each of the above-mentioned alkylene, alkyl, alkenyl, carbocyclyl, heterocyclyl, aryl, heteroaryl and aralkyl groups at each occurrence is optionally substituted with one or more substituents independently selected from halogen, C₁₋₆ alkyl and -OR⁵;
R⁵ and R⁶ at each occurrence are each independently selected from H, C₁₋₆ alkyl, C₃₋₁₀ carbocyclyl, 3-10 membered heterocyclyl, C₆₋₁₀ aryl, 5-14 membered heteroaryl and C₆₋₁₂ aralkyl;
or pharmaceutically acceptable salts, esters, stereoisomers, polymorphs, solvates, N-oxides, isotopically labeled derivatives, metabolites or prodrugs thereof.

The present disclosure also provides a preparation method of the nanocrystal formulation, which includes grinding the ROCK2 inhibitor and a stabilizer.

Another object of the present disclosure is a method and use of the nanocrystal formulation in preventing, alleviating and/or treating idiopathic pulmonary fibrosis.

Other objects of the present disclosure will be apparent to those skilled in the art from the context and examples.

### BRIEF DESCRIPTION OF DRAWINGS

In order to explain the examples of the present disclosure and the technical solutions of the prior art more clearly, the drawings needed to be used in the examples and the prior art are briefly introduced below. Obviously, the drawings in the following description represent only some examples of the present disclosure. For those of ordinary skill in the art, other drawings can also be obtained based on these drawings without exerting creative efforts.
FIGs. 1 and 2 show the dissolution curves of Comparative Examples 1-3 of the present disclosure;
FIG. 3 shows the dissolution curves of Examples 8-9 and the Comparative Example of the present disclosure;
FIGs. 4 to 6 are respectively the dissolution curves of the nanosuspension of Example 8, the nanocrystal tablets of Examples 10-15 and the Comparative Example of the present disclosure;
FIG. 7 shows the dissolution curves of the nanosuspension of Example 8, the nanocrystal tablets of Example 15, the nanocrystal capsules of Examples 24 to 25 and the Comparative Example of the present disclosure.

### Definition

Unless otherwise defined below, all technical and scientific terms used herein are intended to have the same meaning as commonly understood by one of ordinary skill in the art. References to technologies as used herein are intended to refer to technologies as commonly understood in the art, including those technical variations or equivalent technologies that would be apparent to those skilled in the art. Although it is believed that the following terms are well understood by those skilled in the art, the following definitions are set forth to better explain the present disclosure.

The term "nanocrystal" refers to both nanocrystals and nanosuspensions, which represents a stable colloidal dispersion system formed by dispersing nanoscale drug particles in water in the presence of a stabilizer.

The terms "include", "comprise", "have", "contain", or "involve" and their other variations herein are inclusive or open-ended, and other unlisted elements or method steps are not excluded.

As used herein, the term "alkylene" refers to a saturated divalent hydrocarbon group, alternatively a saturated divalent hydrocarbon group having 1, 2, 3, 4, 5 or 6 carbon atoms, such as methylene, ethylene, propylene or butylene.

As used herein, the term "alkyl" is defined as a linear or branched saturated aliphatic hydrocarbon. In some embodiments, an alkyl group has 1 to 12, such as 1 to 6 carbon atoms. For example, as used herein, the term "C₁₋₆ alkyl" refers to a linear or branched group of 1 to 6 carbon atoms (e.g., methyl, ethyl, n-propyl, isopropyl, n-butyl, isobutyl, sec-butyl, tert-butyl, n-pentyl, isopentyl, neopentyl or n-hexyl), which is optionally substituted by 1 or more (such as 1 to 3) suitable substituents such as halogen (in this case, the group is called "haloalkyl") (for example, CH₂F, CHF₂, CF₃, CCl₃, C₂F₅, C₂Cl₅, CH₂CF₃, CH₂Cl or -CH₂CH₂CF₃, etc.). The term "C₁₋₄ alkyl" refers to a linear or branched aliphatic hydrocarbon chain of 1 to 4 carbon atoms (i.e., methyl, ethyl, n-propyl, isopropyl, n-butyl, isobutyl, sec-butyl or tert-butyl).

As used herein, the term "alkenyl" means a linear or branched monovalent hydrocarbon group containing one double bond and having 2 to 6 carbon atoms ("C₂₋₆ alkenyl"). The alkenyl group is, for example, vinyl, 1-propenyl, 2-propenyl, 2-butenyl, 3-butenyl, 2-pentenyl, 3-pentenyl, 4-pentenyl, 2-hexenyl, 3-hexenyl, 4-hexenyl, 5-hexenyl, 2-methyl-2-propenyl and 4-methyl-3-pentenyl. When the compounds of the present disclosure contain an alkenylene group, the compounds may exist in pure E (entgegen) form, pure Z (zusammen) form, or any mixture thereof.

As used herein, the term "alkynyl" means a monovalent hydrocarbon group containing one or more triple bonds, alternatively having 2, 3, 4, 5 or 6 carbon atoms, such as ethynyl or propynyl.

As used herein, the term "cycloalkyl" refers to a saturated monocyclic or polycyclic (such as bicyclic) hydrocarbon ring (e.g., monocyclic rings such as cyclopropyl, cyclobutyl, cyclopentyl, cyclohexyl, cycloheptyl, cyclooctyl, cyclononyl, or bicyclic rings, including spiro, fused or bridged systems (such as bicyclo[1.1.1]pentyl, bicyclo[2.2.1]heptyl, bicyclo[3.2.1]octyl or bicyclo[5.2.0]nonyl, decahydronaphthyl, etc.)), which is optionally substituted by one or more (such as 1 to 3) suitable substituents. The cycloalkyl group has 3 to 15 carbon atoms. For example, the term "C₃₋₆ cycloalkyl" refers to a saturated monocyclic or polycyclic (such as bicyclic) hydrocarbon ring having 3 to 6 ring-forming carbon atoms (such us, cyclopropyl, cyclobutyl, cyclopentyl or cyclohexyl), which is optionally substituted by one or more (such as 1 to 3) suitable substituents, for example methyl-substituted cyclopropyl.

As used herein, the terms "carbocyclylene", "carbocyclyl" and "carbocycle" refer to saturated (i.e., "cycloalkylene" and "cycloalkyl") or unsaturated (i.e., having one or more double and/or triple bonds within a ring) monocyclic or polycyclic hydrocarbon rings having, for example, 3 to 10 (alternatively 3 to 8, yet alternatively 3 to 6) ring carbons, including but not limited to cyclopropyl(ene) (ring), cyclobutyl(ene) (ring), cyclopentyl(ene) (ring), cyclohexyl(ene) (ring), cycloheptyl(ene) (ring), cyclooctyl(ene) (ring), cyclononyl(ene) (ring), cyclohexenyl(ene) (ring), etc.

As used herein, the terms "heterocyclyl", "heterocyclylene" and "heterocycle" refer to saturated (i.e., heterocycloalkyl) or partially unsaturated (i.e., having one or more double and/or triple bonds within a ring) cyclic group having, for example, 3-10 (alternatively 3-8, yet alternatively 3-6) ring atoms, in which at least one ring atom is a heteroatom selected from N, O, and S and the remaining ring atom(s) is C. For example, a "3-10 membered heterocycle(yl)(ene)" is a saturated or partially unsaturated heterocycle(yl)(ene) having 2-9 (such as 2, 3, 4, 5, 6, 7, 8 or 9) ring carbon atoms and one or more (such as 1, 2, 3 or 4) heteroatoms independently selected from N, O and S. Examples of heterocyclylene and heterocycle(yl) include, but are not limited to: oxiranyl(ene), aziridinyl(ene), azetidinyl(ene), oxetanyl(ene), tetrahydrofuranyl(ene), dioxolinyl(ene), pyrrolidinyl(ene), pyrrolidonyl(ene), imidazolidinyl(ene), pyrazolidinyl(ene), pyrrolinyl(ene), tetrahydropyranyl(ene), piperidinyl(ene), morpholinyl(ene), dithianyl(ene), thiomorpholinyl(ene), piperazinyl(ene) or trithianyl(ene). The groups also encompass bicyclic systems, including spiro, fused or bridged systems (such as 8-azaspiro[4.5]decane, 3,9-diazaspiro[5.5]undecane, 2-azabicyclo[2.2.2]octane, etc.). Heterocyclylene and heterocycle(yl) may be optionally substituted with one or more (such as 1, 2, 3 or 4) suitable substituents.

As used herein, the terms "aryl(ene)" and "aromatic ring" refer to all-carbon monocyclic or fused polycyclic aromatic radicals having a conjugated pi electron system. For example, as used herein, the terms "C₆₋₁₀ aryl(ene)" and "C₆₋₁₀ aromatic ring" refer to aromatic groups containing 6 to 10 carbon atoms, such as phenyl(ene)(benzene ring) or naphthyl(ene) (naphthalene ring). The aryl(ene) and aromatic ring are optionally substituted with one or more (such as 1 to 3) suitable substituents (e.g., halogen, -OH, -CN, -NO₂, C₁₋₆ alkyl, etc.).

As used herein, the terms "heteroaryl(ene)" and "heteroaryl ring" refer to a monocyclic, bicyclic or tricyclic aromatic ring system having 5, 6, 8, 9, 10, 11, 12, 13 or 14 ring atoms, especially 1 or 2 or 3 or 4 or 5 or 6 or 9 or 10 carbon atoms, which comprises at least one heteroatom (for example oxygen, nitrogen or sulfur) which may be the same or different, and in each case, may be benzo-fused. In particular, "heteroaryl(ene)" or "heteroaryl ring" is selected from thienyl(ene), furyl(ene), pyrrolyl(ene), oxazolyl(ene), thiazolyl(ene), imidazolyl(ene), pyrazolyl(ene), isoxazolyl(ene), isothiazolyl(ene), oxadiazolyl(ene), triazolyl(ene), thiadiazolyl(ene), etc., and their benzo derivatives; or pyridyl(ene), pyridazinyl(ene), pyrimidinyl(ene), pyrazinyl(ene), triazinyl(ene), etc., and their benzo derivatives.

As used herein, the term "aralkyl" alternatively means an aryl- or heteroaryl-substituted alkyl group, wherein the aryl, heteroaryl and alkyl are as defined herein. Typically, the aryl group may have 6 to 14 carbon atoms, the heteroaryl group may have 5 to 14 ring atoms, and the alkyl group may have 1 to 6 carbon atoms. Exemplary aralkyl groups include, but are not limited to, benzyl, phenylethyl, phenylpropyl, and phenylbutyl.

As used herein, the term "halo" or "halogen" group is defined to include F, Cl, Br or I.

As used herein, the term "nitrogen-containing heterocycle" refers to a saturated or unsaturated monocyclic or bicyclic group having 2, 3, 4, 5, 6, 7, 8, 9, 10, 11, 12 or 13 carbon atoms and at least one nitrogen atom in the ring, which may also optionally contain one or more (such as one, two, three or four) ring members selected from N, O, C=O, S, S=O and S(=O)₂, which are connected to the rest of the molecule through the nitrogen atom and any remaining ring atom in the nitrogen-containing heterocycle. The nitrogen-containing heterocyclic ring is optionally benzofused, and is alternatively connected to the rest of the molecule through the nitrogen atom in the nitrogen-containing heterocyclic ring and any carbon atom in the fused benzene ring.

The term "substituted" means that one or more (e.g., one, two, three or four) hydrogens on the specified atom are replaced by the selection from the specified group, provided that the normal valence of the specified atom in the current situation is not exceeded and the substitution forms a stable compound. Combinations of substituents and/or variables are permissible only if such combinations form stable compounds.

If a substituent is described as "optionally substituted", the substituent may be (1) unsubstituted or (2) substituted. If the carbon of a substituent is described as being optionally substituted by one or more in the substituent lists, then one or more hydrogens (to the extent of any hydrogen present) on the carbon can be independently and/or collectively replaced by independently selected optional substituents. If the nitrogen of a substituent is described as being optionally substituted by one or more in the substituents listed, then one or more hydrogens (to the extent of any hydrogen present) on the nitrogen each can be replaced by an independently selected optional substituents.

If a substituent is described as being "independently selected from", each substituent is selected independently of the other. Therefore, each substituent may be the same as or different from another (other) substituent.

As used herein, the term "one or more" means one or more than 1, such as 2, 3, 4, 5 or 10 under reasonable conditions.

Unless otherwise specified, as used herein, the point of attachment of a substituent can be from any suitable position on the substituent.

When the bond of a substituent is shown as crossing a bond connecting two atoms in a ring, then such substituent can be bonded to any substitutable ring-forming atom in the ring.

The present disclosure also includes all pharmaceutically acceptable isotopically labeled compounds that are identical to the compounds of the present disclosure except that one or more atoms are replaced by atoms with the same atomic number but the atomic mass or mass number different from that prevailing in nature. Examples of isotopes suitable for inclusion in the compounds of the present disclosure include, but are not limited to, isotopes of hydrogen (e.g., deuterium (²H), tritium (³H)); isotopes of carbon (e.g.,¹¹C, ¹³C, and ¹⁴C); isotopes of chlorine (e.g. ³⁶Cl); isotopes of fluorine (e.g. ¹⁸F); isotopes of iodine (e.g. ¹²³I and ¹²⁵I); isotopes of nitrogen (e.g. ¹³N and ¹⁵N); isotopes of oxygen (e.g. ¹⁵O, ¹⁷O and ¹⁸O); isotopes of phosphorus (e.g. ³²P); and isotopes of sulfur (e.g. ³⁵S). Some isotopically labeled compounds of the present disclosure (e.g., those incorporating radioactive isotopes) can be used in drug and/or substrate tissue distribution studies (e.g., assays). The radioactive isotopes tritium (i.e. ³H) and carbon-14 (i.e. ¹⁴C) are particularly useful for this purpose because of their easy incorporation and easy detection. Substitution with positron emitting isotopes (such as ¹¹C, ¹⁸F, ¹⁵O and ¹³N) can be used to test substrate receptor occupancy in positron emission tomography (PET) studies. Isotopically labeled compounds of the present disclosure can be prepared by using appropriate isotopically labeled reagents in place of the previously employed unlabeled reagents by methods similar to those described in the accompanying Schemes and/or Examples and Preparation. Pharmaceutically acceptable solvates of the present disclosure include those in which the crystallization solvent can be isotopically substituted, for example, D₂O, acetone-d₆ or DMSO-d₆.

The term "stereoisomer" means an isomer formed due to at least one asymmetric center. In compounds with one or more (e.g., one, two, three or four) asymmetric centers, they can give rise to racemic mixtures, single enantiomers, diastereomeric mixtures and individual diastereomers. Specific individual molecules can also exist as geometric isomers (cis/trans). Similarly, the compounds of the present disclosure can exist as a mixture of two or more structurally different forms in rapid equilibrium (often referred to as tautomers). Representative examples of tautomers include keto-enol tautomers, phenol-ketone tautomers, nitroso-oxime tautomers, and imine-enamine tautomers, etc. It is understood that the scope of the present disclosure encompasses all such isomers or mixtures thereof in any proportion (e.g., 60%, 65%, 70%, 75%, 80%, 85%, 90%, 95%, 96%, 97%, 98%, 99%).

Chemical bonds of the compounds of the present disclosure can be depicted herein using solid lines ( 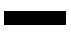 ), solid wedges ( 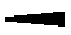 ), or dashed wedges ( 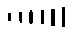 ). The use of a solid line to depict a bond to an asymmetric carbon atom is intended to indicate that all possible stereoisomers at that carbon atom are included (e.g., a specific enantiomer, a racemic mixture, etc.). The use of solid or dashed wedges to depict bonds to asymmetric carbon atoms is intended to indicate that the shown stereoisomers exist. When present in a racemic mixture, solid and dashed wedges are used to define relative stereochemistry rather than absolute stereochemistry. Unless otherwise specified, the compounds of the present disclosure are intended to exist in the form of stereoisomers, including cis- and trans-isomers, optical isomers (e.g., R and S enantiomers), diastereomers, geometric isomers, rotamers, conformational isomers, atropisomers and mixtures thereof). The compounds of the present disclosure can exhibit more than one type of isomerisms and are composed of mixtures thereof (e.g., racemic mixtures and diastereomer pairs).

The present disclosure encompasses all possible crystalline forms or polymorphs of the compounds of the present disclosure, which can be a single polymorph or a mixture of more than one polymorph in any proportion.

It should also be understood that some compounds of the present disclosure may exist in free form for therapeutic use, or, where appropriate, as pharmaceutically acceptable derivatives thereof. In the present disclosure, pharmaceutically acceptable derivatives include, but are not limited to, pharmaceutically acceptable salts, esters, solvates, N-oxides, metabolites or prodrugs, which can directly or indirectly provide the compounds of the present disclosure or its metabolites or residues after being administered to patients in need thereof. Therefore, when reference is made herein to "compounds of the present disclosure", it is also intended to encompass the various derivative forms of the compounds described above.

Pharmaceutically acceptable salts of the compounds of the present disclosure include acid addition salts and base addition salts thereof.

Suitable acid addition salts are formed from acids that form pharmaceutically acceptable salts. Examples include acetate, adipate, aspartate, benzoate, benzenesulfonate, bicarbonate/carbonate, bisulfate/sulfate, borate, camphorsulfonate, citrate, cyclamate, ethanedisulfonate, ethanesulfonate, formate, fumarate, glucoheptonate, gluconate, glucuronate, hexafluorophosphate, hypobenate, hydrochloride/chloride, hydrobromide/bromide, hydroiodide/iodide, isethionate, lactate, malate, maleate, malonate, methanesulfonate, methylsulfate, naphthylate, 2-naphthalenesulfonate, nicotinate, nitrate, orotate, oxalate, palmitate, pamoate, phosphate/hydrogen phosphate/dihydrogen phosphate, pyroglutamate, saccharate, stearate, succinate, tannins, tartrate, toluenesulfonate, trifluoroacetate and xinafoate.

Suitable base addition salts are formed from bases that form pharmaceutically acceptable salts. Examples include aluminum salts, arginine salts, benzathine penicillin salts, calcium salts, choline salts, diethylamine salts, diethanolamine salts, glycinate salts, lysine salts, magnesium salts, meglumine salts, ethanolamine salts, potassium salts, sodium salts, tromethamine salts and zinc salts.

For a review of suitable salts, see Stahl and Wermuth, "Handbook of Pharmaceutical Salts: Properties, Selection, and Use" (Wiley-VCH, 2002). Methods for preparing pharmaceutically acceptable salts of the compounds of the present disclosure are known to those skilled in the art.

As used herein, the term "ester" means esters derived from the compounds of each general formula herein, including physiologically hydrolyzable esters (which can be hydrolyzed under physiological conditions to release the free acid or alcohol form of the compounds of the present disclosure). The compounds of the present disclosure themselves may also be esters.

The compounds of the present disclosure may exist in the form of solvates, alternatively hydrates, wherein the compounds of the present disclosure comprise a polar solvent as structural elements of the crystal lattice of the compounds, in particular such as water, methanol or ethanol. The amount of a polar solvent, especially water, may be present in a stoichiometric or non-stoichiometric ratio.

Those skilled in the art will understand that not all nitrogen-containing heterocycles are capable of forming N-oxides because nitrogen requires an available lone pair of electrons to be oxidized into an oxide; those skilled in the art will recognize nitrogen-containing heterocycles capable of forming N-oxides. Those skilled in the art will also recognize that tertiary amines are capable of forming N-oxides. The synthetic methods for the preparation of N-oxides of heterocyclic and tertiary amines are well known to those skilled in the art and include the use of peroxyacids such as peracetic acid and m-chloroperoxybenzoic acid (MCPBA), hydrogen peroxide, alkyl hydroperoxides such as tert-butyl hydroperoxide, sodium perborate and dioxirane such as dimethyldioxirane to oxidize heterocyclic and tertiary amines. These methods for preparing N-oxides have been extensively described and reviewed in the literature, see for example: T. L. Gilchrist, Comprehensive Organic Synthesis, vol.7, pp 748-750; A. R. Katritzky and A. J. Boulton, Eds., Academic Press; and G. W. H. Cheeseman and E. S. G. Werstiuk, Advances in Heterocyclic Chemistry, vol.22, pp 390-392, A. R. Katritzky and A. J. Boulton, Eds., Academic Press.

Also included within the scope of the present disclosure are metabolites of the compounds of the present disclosure, that is, substances formed in vivo upon administration of the compounds of the present disclosure. Such products can be produced by, for example, oxidation, reduction, hydrolysis, amidation, deamidation, esterification, enzymolysis, etc. of the administered compound. Accordingly, the present disclosure includes metabolites of the compounds of the present disclosure, including compounds prepared by contacting a compound of the present disclosure with a mammal for a time sufficient to produce metabolites thereof.

The present disclosure further includes, within its scope, prodrugs of the compounds of the present disclosure, which are certain derivatives of the compounds of the present disclosure which may themselves have little or no pharmacological activity and can be converted into the compounds of the present disclosure with desired activity by, for example, hydrolytic cleavage when administered into or on the body. Generally, such prodrugs will be functional group derivatives of the compound that are readily converted in vivo to the desired therapeutically active compound. Additional information on the use of prodrugs can be found in "Pro-drugs as Novel Delivery Systems", Volume 14, ACS Symposium Series (T. Higuchi and V. Stella). The prodrugs of the present disclosure may be prepared, for example, by replacing the appropriate functional groups present in the compounds of the present disclosure with certain moieties known to those skilled in the art as "pro-moiety" (such as, as described in "Design of Prodrugs", H. Bundgaard (Elsevier, 1985)).

The present disclosure also encompasses compounds of the present disclosure containing protecting groups. In any process for preparing the compounds of the present disclosure, it may be necessary and/or desirable to protect sensitive groups or reactive groups on any relevant molecules, thereby forming a chemically protected forms of the compounds of the present disclosure. This can be accomplished by conventional protecting groups, such as those described in TW Greene & PGM Wuts, Protective Groups in Organic Synthesis, John Wiley & Sons, 1991, which references are incorporated herein by reference. The protecting groups can be removed at an appropriate subsequent stage using methods known in the art.

The term "about" means within ±10%, alternatively within ±5%, yet alternatively within ±2% of the stated value.

The term "effective amount" refers to an amount sufficient to achieve a desired therapeutic effect under the conditions of administration, leading to pathological symptoms, disease progression, improvement of physiological conditions associated therewith or induction of resistance to the progression of the aforementioned diseases.

Unless otherwise stated, the term "treating" as used herein means reversing, alleviating, inhibiting the disorder or condition to which such term applies or the progression of one or more symptoms of such disorder or condition, or preventing such disease or condition or one or more symptoms of such disease or condition.

As used herein, "individual" includes humans or non-human animals. Exemplary human individuals include human individuals (referred to as patients) suffering from a disease, such as those described herein, or normal individuals. In the present disclosure, "non-human animals" include all vertebrates, such as non-mammals (such as birds, amphibians, reptiles) and mammals, such as non-human primates, domestic animals and/or domesticated animals (such as sheep, dogs, cats, cows, pigs, etc.).

### DETAILED DESCRIPTION

In one embodiment, the present disclosure relates to a nanocrystal formulation comprising a ROCK2 inhibitor and a stabilizer.

In one embodiment, the present disclosure relates to a nanocrystal formulation, wherein the ROCK2 inhibitor is a compound of formula (I), 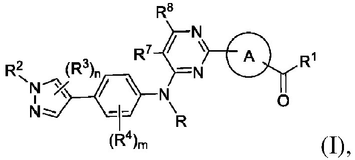 wherein
Ring A is 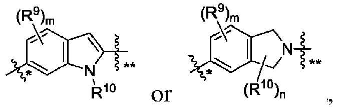 wherein the above group is connected to the pyrimidine ring through one of the two positions marked by * or **, and the other position is connected to the carbonyl group;
R⁹ and R¹⁰ at each occurrence are each independently selected from H, halogen, C₁₋₆ alkyl, C₂₋₆ alkenyl, C₃₋₁₀ carbocyclyl, 3-10 membered heterocyclyl, C₆₋₁₀ aryl, 5-14 membered heteroaryl, C₆₋₁₂ aralkyl, -C(=O)R⁵ and -C₁₋₆ alkylene-O(P=O)(OH)₂;
m at each occurrence is independently an integer of 0, 1, 2, or 3; and
n at each occurrence is independently an integer of 0, 1 or 2;
alternatively, ring A is 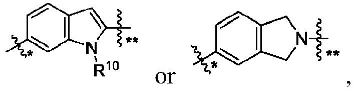 wherein the above group is connected to the pyrimidine ring through the position marked by *, and is connected to the carbonyl group through the position marked by **, wherein R¹⁰ is selected from H and C₁₋₆ alkyl, alternatively H or methyl;
R is selected from H and C₁₋₆ alkyl;
R¹ is 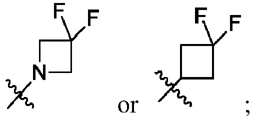
R² is selected from H and C₁₋₆ alkyl;
R³, R⁴, R⁷ and R⁸ at each occurrence are each independently selected from H, halogen, -NR⁵R⁶, -OH, C₁₋₆ alkyl and -OR⁵;
Each of the above-mentioned alkylene, alkyl, alkenyl, carbocyclyl, heterocyclyl, aryl, heteroaryl and aralkyl groups at each occurrence is optionally substituted with one or more substituents independently selected from halogen, C₁₋₆ alkyl and -OR⁵;
R⁵ and R⁶ at each occurrence are each independently selected from H, C₁₋₆ alkyl, C₃₋₁₀ carbocyclyl, 3-10 membered heterocyclyl, C₆₋₁₀ aryl, 5-14 membered heteroaryl and C₆₋₁₂ aralkyl;
or pharmaceutically acceptable salts, esters, stereoisomers, polymorphs, solvates, N-oxides, isotopically labeled derivatives, metabolites or prodrugs thereof.

In one embodiment, the present disclosure relates to a nanocrystal formulation, and the ROCK2 inhibitor is a compound of formula (II) or pharmaceutically acceptable salts, esters, stereoisomers, polymorphs, solvates, N-oxides, isotopically labeled derivatives, metabolites or prodrugs thereof, 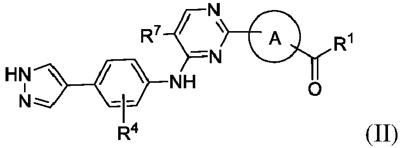 wherein each group is as defined above.

In one embodiment, the present disclosure relates to a nanocrystal formulation, and the ROCK2 inhibitor is a compound of formula (III) or pharmaceutically acceptable salts, esters, stereoisomers, polymorphs, solvates, N-oxides, isotopically labeled derivatives, metabolites or prodrugs thereof, 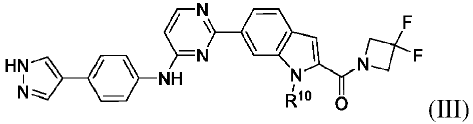 wherein R¹⁰ is H or methyl, alternatively methyl.

In one embodiment, the present disclosure relates to a nanocrystal formulation, and the ROCK2 inhibitor is a compound of formula (IV), or pharmaceutically acceptable salts(especially a hydrochloride, esters, stereoisomers, polymorphs, solvates, N-oxides, isotopically labeled derivatives, metabolites or prodrugs thereof,

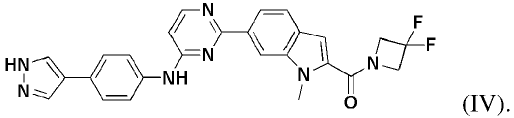

The chemical name of the compound of formula (IV) is: [6-[4-[[4-(1H-pyrazol-4-yl)phenyl]amino]pyrimidin-2-yl]-1-methyl-1H-indole-2-yl](3,3-diflu oroazetidin- 1-yl)methanone.

In the present disclosure, the compound of formula (IV) as a raw material drug can be prepared by known methods or obtained through commercial channels. No matter which method is used to obtain the compound of formula (IV), it is easy to realize for those skilled in the art. Therefore, the present disclosure will not elaborate on the obtainment of the compound of formula (IV).

Generally, a stabilizer needs to be added during the preparation process of nanocrystals. The main reasons are as follows: on the one hand, nanomicronization can significantly increase the specific surface area of drug particles, which also increases the free energy of the entire formulation preparation system, leading to the instability of the system. On the other hand, as the temperature increases due to mechanical energy production during the grinding process, the newly formed nanoparticles will be affected by free energy changes and be re-aggregated and recrystallized. These factors may cause the reduced particle size to return to a larger level, reducing the surface area for dissolution, thereby affecting bioavailability in the body. The stabilizer blocks the re-aggregation of the drug particles by adsorbing on the surface of the drug particles, thereby increasing the effective surface area and significantly improving the stability of a nanosuspension.

In the present disclosure, in addition to improving the wettability of the drug and stabilizing the particle size of the drug, the stabilizer can also improve the dissolution rate of the ROCK2 inhibitor nanocrystals by improving the particle size stability of the ROCK2 inhibitor nanocrystals after they are solidified, dried and reconstituted. Those skilled in the art can select a suitable stabilizer based on the description of the role of a stabilizer in the present disclosure. The stabilizer includes but is not limited to polysorbate 20, polysorbate 40, polysorbate 60, polysorbate 65, polysorbate 80, polysorbate 85, povidone K29/32, polyoxyethylene fatty acid ester, poloxamer 188, poloxamer 407, hydroxypropyl cellulose (HPC), hydroxypropyl methylcellulose (HPMC 3 cps), polyvinylpyrrolidone (PVP K30), poloxamer (Pluronic F68 and Pluronic F127), sodium dodecyl sulfate (SDS), docusate sodium (DSS), polyethylene glycol 15-hydroxystearate, polyoxyethylene castor oil, copovidone, etc.

In one embodiment, the present disclosure relates to a nanocrystal formulation, wherein the stabilizer is selected from one or more of polysorbate, povidone, hydroxypropyl methylcellulose, polyethylene glycol, polyvinyl alcohol, polyoxyethylene castor oil, poloxamer and sodium lauryl sulfate, lactose and mannitol.

In one embodiment, the present disclosure relates to a nanocrystal formulation, wherein the particle size D₉₀ of the nanocrystal formulation is 50-1500nm, alternatively 50-1000nm, alternatively 50-500nm, alternatively 80-300nm, yet alternatively 50nm, 100nm, 150nm, 200nm, 250nm, 300nm, 400nm, 500nm, 600nm, 700nm, 800nm, 900nm or 1000nm.

In one embodiment, the present disclosure relates to a nanocrystal formulation, wherein the particle size range of the ROCK2 inhibitor is alternatively D₉₀ 5~300 µm, alternatively 10-100 µm, and yet alternatively 10-50 µm.

In one embodiment, the present disclosure relates to a nanocrystal formulation, wherein the weight percentage of the ROCK2 inhibitor can be 1%-55%, 4%-50%, 1%-10%, 10%-40%, 10%-35%, 20%-30% or 30%-40%, also can be 1%, 2%, 3%, 4%, 4.5%, 5%, 6%, 7%, 8%, 9%, 9.5%, 10%, 11%, 12%, 13%, 14%, 15%, 15%, 16%, 18%, 19%, 20%, 21%, 22%, 23%, 24%, 25%, 26%, 27%, 28%, 29%, 30%, 31%, 32%, 33%, 34%, 35%, 36%, 37%, 38%, 39% or 40%, based on the total weight of the nanocrystal formulation.

In one embodiment, the present disclosure relates to a nanocrystal formulation, wherein the weight percentage of the stabilizer can be 0.1%-55%, 0.1%-30%, 0.5%-1%, 1%-10%, 10%-20% or 20%-30%, also can be 1%, 2%, 2.5%, 3%, 4%, 5%, 6%, 7%, 8%, 9%, 9.5%, 10%, 11%, 12%, 13%, 14%, 15%, 15%, 16%, 18%, 19%, 20%, 21%, 22%, 23%, 24%, 25%, 26%, 27%, 28%, 29%, 30%, 31%, 32%, 33%, 34%, 35%, 36%, 37%, 38%, 39% or 40%, based on the total weight of the nanocrystal formulation.

In one embodiment, the present disclosure relates to a nanocrystal formulation, wherein the weight ratio of the ROCK2 inhibitor to the stabilizer can be 1:10 to 10:1, 1:9 to 9:1, 1:8 to 8:1. 1:7 to 7:1, 1:6 to 6:1, 1:5 to 5:1, 1:4 to 4:1, 1:3 to 3:1, 1:2 to 2:1, 1:1; can also be 4:1 to 1:1 or 1:1 to 1:2; can also be 10:1, 9:1, 8:1, 7:1, 6:1, 5:1, 4:1, 3:1, 2:1, 1:1, 1:2, 1:3, 5:4, 5:3 or 5:2.

In one embodiment, the present disclosure relates to a nanocrystal formulation further containing an excipient.

In one embodiment, the present disclosure relates to a nanocrystal formulation, wherein the excipient is selected from one or more of fillers; wetting agents; sweeteners or flavoring agents; surfactants; binders; disintegrants; lubricants; glidants or anti-adhesive agents; release modifiers; coating agents; emulsifiers; solubilizers; and fragrances.

In one embodiment, the present disclosure relates to a nanocrystal formulation, wherein the excipient includes a filler; the filler can improve the material properties of an active ingredient, improve the stickiness and electrostatic properties, thereby facilitating subsequent shaping of the composition, such as tableting, filling capsules and the like, and play an important role in the preparation of solid formulations. Fillers can also adjust the dissolution rate of a formulation. In the technical solution of the present disclosure, fillers commonly used in the technical field can be selected, including but not limited to one or at least two of microcrystalline cellulose, mannitol, lactose, starch, pregelatinized starch, dextrin, calcium phosphate dihydrate, and anhydrous calcium hydrogen phosphate. In some embodiments of the present disclosure, based on the total weight of the nanocrystal formulation, the weight percentage of the filler can be 1%-80%; more specifically, in some embodiments, the weight percentage of the filler can be 20%-70%, 30%-60% or 50-70%, etc., such as 20%, 25%, 30%, 35%, 40%, 45%, 50%, 55%, 60%, 65% or 70%, etc. In some specific embodiments of the present disclosure, the filler is selected from one or more of microcrystalline cellulose, lactose, and mannitol, alternatively, the filler is mannitol.

In one embodiment, the present disclosure relates to a nanocrystal formulation, wherein the excipient includes lubricants, which facilitate various processing steps including component mixing, tableting, etc.; for example, the lubricants can make the pressure distribution uniform during tableting and make the density of tablets uniform; and reduce the force required to push the tablet out of the die hole. Another possible function of lubricants is to improve the appearance of the tablet, making the surface of the tablet smooth and flat. In the technical solution of the present disclosure, lubricants commonly used in the technical field can be selected, including but not limited to one or a combination of at least two of magnesium stearate, talc powder, micronized silica gel, sodium stearyl fumarate, glyceryl behenate and polyethylene glycol, yet alternatively magnesium stearate. In some embodiments of the present disclosure, based on the total weight of the nanocrystal formulation, the weight percentage of the lubricant can be 0.1% to 5%, 0.1%-1.5%, or 0.5%-1%, etc., such as 0.1%, 0.2%, 0.3%, 0.4%, 0.5%, 0.6%, 0.7%, 0.8%, 0.9%, 1.0%, 1.1%, 1.2%, 1.3%, 1.4% or 1.5%, etc. The lubricant is selected from magnesium stearate, talc powder, micronized silica gel, sodium stearyl fumarate, glyceryl behenate and polyethylene glycol.

In one embodiment, the present disclosure relates to a nanocrystal formulation, wherein the excipient includes a disintegrant. In the technical solution of the present disclosure, disintegrants commonly used in the technical field can be selected, including but not limited to one or more of croscarmellose sodium and crospovidone, etc. In some embodiments of the present disclosure, based on the total weight of the nanocrystal formulation, the weight percentage of the disintegrant may be 0 to 20%, alternatively 0 to 10%, and yet alternatively 2 to 10%, such as 3%, 4%, 5%, 6%, 7%, 8%, 9% or 10%.

In one embodiment, the present disclosure relates to a nanocrystal formulation wherein the excipient includes a glidant. In the technical solution of the present disclosure, glidants commonly used in the technical field can be selected, including but not limited to silicon dioxide, etc. In some embodiments of the present disclosure, based on the total weight of the nanocrystal formulation, the weight percentage of the glidant may be 0 to 20%, alternatively 0 to 15%, and yet alternatively 2 to 12%, such as 2%, 2.5%, 3%, 4%, 5%, 6%, 7%, 8%, 9%, 10% or 11%.

In one embodiment, the present disclosure relates to a nanocrystal formulation, wherein the nanocrystal formulation further comprises a solvent. In the technical solution of the present disclosure, solvents commonly used in the technical field can be selected, including but not limited to water, etc., and purified water is alternative. In some embodiments of the present disclosure, based on the total weight of the nanocrystal formulation, the weight percentage of the solvent may be 0 to 99%, alternatively 80 to 99%, and yet alternatively 85% to 95%, such as 86%, 87%, 88%, 89%, 90%, 91%, 92%, 93%, 94% or 95%.

In one embodiment, the present disclosure relates to a nanocrystal formulation, wherein the nanocrystal formulation further comprises a bacteriostatic agent. In the technical solution of the present disclosure, bacteriostatic agents commonly used in the technical field can be selected, including but not limited to one or more of methylparaben, propylparaben, and the like. In some embodiments of the present disclosure, based on the total weight of the nanocrystal formulation, the weight percentage of the bacteriostatic agent can be 0 to 5%, alternatively 0 to 1%, yet alternatively 0.01% to 0.5%, such as 0.1%, 0.2%, 0.3%, 0.4% or 0.5%.

In one embodiment, the present disclosure relates to a nanocrystal formulation, wherein the nanocrystal formulation is selected from suspensions, tablets, capsules, granules, powders, lozenges and pills; alternatively suspensions, tablets or capsules.

In one embodiment, the present disclosure relates to a nanocrystal formulation, wherein the nanocrystal formulation is a suspension, comprising:
- 1-10% of ROCK2 inhibitor, alternatively 1%, 2%, 3%, 4%, 4.5%, 5%, 6%, 7%, 8%, 9% or 10% of ROCK2 inhibitor, yet alternatively 4%, 4.5%, or 5% of ROCK2 inhibitor; and
- 1-10% of stabilizer, alternatively 1%, 1.5%, 2%, 2.5%, 5%, 9%, 9.5% or 10% of stabilizer.

In one embodiment, the present disclosure relates to a nanocrystal formulation, wherein the nanocrystal is a suspension, comprising 11.13g of ROCK2 inhibitor, 0.77g of polysorbate 80, 5.00g of povidone K29/32 and 233.10 g of purified water, alternatively, the particle size of the ROCK2 inhibitor is 50-1000nm, alternatively 50-500nm, yet alternatively 50-300nm.

In one embodiment, the present disclosure relates to a nanocrystal formulation, wherein the nanocrystal is a suspension, comprising 11.13g of ROCK2 inhibitor, 0.77g of polysorbate 80, 2.50g of povidone K29/32 and 235.60 g of purified water, alternatively, the particle size of the ROCK2 inhibitor is 50-1000nm, alternatively 50-500nm, yet alternatively 50-300nm.

In one embodiment, the present disclosure relates to a nanocrystal formulation, wherein the nanocrystal is a suspension, comprising 11.13g of ROCK2 inhibitor, 0.77g of polysorbate 80, 2.50g of hypromellose and 235.60g of purified water, alternatively, the particle size of the ROCK2 inhibitor is 50-1000nm, alternatively 50-500nm, yet alternatively 50-300nm.

In one embodiment, the present disclosure relates to a nanocrystal formulation, wherein the nanocrystal is a suspension, comprising 11.13g of ROCK2 inhibitor, 0.77g of polysorbate 80, 5.00g of hypromellose and 233.10g of purified water, alternatively, the particle size of the ROCK2 inhibitor is 50-1000nm, alternatively 50-500nm, yet alternatively 50-300nm.

In one embodiment, the present disclosure relates to a nanocrystal formulation, wherein the nanocrystal is a suspension, comprising 250.08g of ROCK2 inhibitor, 17.40g of polysorbate 80, 111.60g of povidone K29/32, 10.00 g of methylparaben, 1.10 g of propylparaben and 5189.82g of purified water, alternatively, the particle size of the ROCK2 inhibitor is 50-1000 nm, alternatively 50-500 nm, yet alternatively 50-300 nm.

In one embodiment, the present disclosure relates to a nanocrystal formulation, wherein the nanocrystal is a suspension, comprising 55.19g of ROCK2 inhibitor, 18.61g of polysorbate 80, 99.25g of polyoxyethylene castor oil, 2.23g of methylparaben, 0.25g of propylparaben and 1065.09g of purified water, alternatively, the particle size of the ROCK2 inhibitor is 50-1000nm, alternatively 50-500nm, yet alternatively 50-300nm, still alternatively 50-150nm.

In one embodiment, the present disclosure relates to a nanocrystal formulation, wherein the nanocrystal formulation is a tablet, comprising:
- 10-30% of ROCK2 inhibitor, alternatively 20-30% of ROCK2 inhibitor, alternatively 20%, 22%, 25%, 28% or 30% of ROCK2 inhibitor;
- 1-20% of stabilizer, alternatively 5-20% of stabilizer, yet alternatively 5%, 8%, 10%, 13%, 15%, 18% or 20% of stabilizer.

In one embodiment, the present disclosure relates to a nanocrystal formulation, wherein the nanocrystal is a tablet, comprising 11.70g of ROCK2 inhibitor, 3.15g of polysorbate 80, 5.26g of lactose, 1.05g of polyethylene glycol 6000, 16.03g of mannitol, 4.94g of silicon dioxide, 2.47g of sodium lauryl sulfate, 2.96g of microcrystalline cellulose, 2.96g of croscarmellose sodium and 0.32g of magnesium stearate, alternatively, the particle size of the ROCK2 inhibitor is 50-1000nm, alternatively 50-500nm, yet alternatively 50-300nm, still alternatively 50-150nm.

In one embodiment, the present disclosure relates to a nanocrystal formulation, wherein the nanocrystal is a tablet, comprising 32.71g of ROCK2 inhibitor, 14.81g of polysorbate 80, 14.71g of lactose, 2.94g of polyethylene glycol 6000, 43.63g of mannitol, 16.00g of silicon dioxide, 8.00g of sodium lauryl sulfate, 12.80g of microcrystalline cellulose, 12.80g of croscarmellose sodium and 1.60g of sodium stearyl fumarate, alternatively, the particle size of the ROCK2 inhibitor is 50-1000nm, alternatively 50-500nm, yet alternatively 50-300nm, still alternatively 50-150nm.

In one embodiment, the present disclosure relates to a nanocrystal formulation, wherein the nanocrystal is a tablet, comprising 3.34g of ROCK2 inhibitor, 0.89g of polysorbate 80, 1.80g of lactose, 0.30g of polyethylene glycol 6000, 0.75g of silicon dioxide, 0.75g of sodium lauryl sulfate, 5.82g of microcrystalline cellulose, 1.20g of croscarmellose sodium and 0.15g of sodium stearyl fumarate, alternatively, the particle size of the ROCK2 inhibitor is 50-1000nm, alternatively 50-500nm, yet alternatively 50-300nm, still alternatively 50-150nm.

In one embodiment, the present disclosure relates to a nanocrystal formulation, wherein the nanocrystal is a tablet, comprising 3.34g of ROCK2 inhibitor, 0.89g of polysorbate 80, 1.80g of lactose, 0.30g of polyethylene glycol 6000, 0.75g of silicon dioxide, 0.45g of sodium lauryl sulfate, 4.50g of microcrystalline cellulose, 1.62g of pregelatinized starch, 1.20g of croscarmellose sodium and 0.15g of sodium stearyl fumarate, alternatively, the particle size of the ROCK2 inhibitor is 50-1000 nm, alternatively 50-500 nm, yet alternatively 50-300 nm, still alternatively 50-150 nm.

In one embodiment, the present disclosure relates to a nanocrystal formulation, wherein the nanocrystal is a tablet, comprising 11.14g of ROCK2 inhibitor, 2.97g of polysorbate 80, 6.01g of lactose, 1.00g of polyethylene glycol 6000, 1.00g of silicon dioxide, 27.38g of spray-dried mannitol and 0.50g of sodium stearyl fumarate, alternatively, the particle size of the ROCK2 inhibitor is 50-1000nm, alternatively 50-500nm, yet alternatively 50-300nm, still alternatively 50-150nm.

In one embodiment, the present disclosure relates to a nanocrystal formulation, wherein the nanocrystal is a tablet, comprising 111.20g of ROCK2 inhibitor, 30.04g of polysorbate 80, 59.99g of lactose, 10.00g of polyethylene glycol 6000, 10.00g of silicon dioxide, 273.78g of spray-dried mannitol and 5.00g of sodium stearyl fumarate, alternatively, the particle size of the ROCK2 inhibitor is 50-1000nm, alternatively 50-500nm, yet alternatively 50-300nm, still alternatively 50-150nm.

In one embodiment, the present disclosure relates to a nanocrystal formulation, wherein the nanocrystal formulation is a tablet, comprising:
- 10-30% of ROCK2 inhibitor, alternatively 20-30% of ROCK2 inhibitor, yet alternatively 20%, 22%, 25%, 28% or 30% of ROCK2 inhibitor;
- 10-30% of stabilizer, alternatively 20-30% of stabilizer, yet alternatively 20%, 22%, 25%, 28% or 30% of stabilizer.

In one embodiment, the present disclosure relates to a nanocrystal formulation, wherein the nanocrystal is a tablet, comprising 22.22g of ROCK2 inhibitor, 20.00g of polysorbate 80, 54.80g of mannitol, 2.00g of silicon dioxide and 1.00g of sodium stearyl fumarate, alternatively, the particle size of the ROCK2 inhibitor is 50-1000nm, alternatively 50-500nm, yet alternatively 50-300nm, still alternatively 50-150nm.

In one embodiment, the present disclosure relates to a nanocrystal formulation, wherein the nanocrystal is a tablet, comprising 22.20g of ROCK2 inhibitor, 10.00g of polysorbate 80, 20.00g of povidone K29/32, 44.80g of mannitol, 2.00g of silicon dioxide and 1.00g of sodium stearyl fumarate, alternatively, the particle size of the ROCK2 inhibitor is 50-1000nm, alternatively 50-500nm, yet alternatively 50-300nm, still alternatively 50-150nm.

In one embodiment, the present disclosure relates to a nanocrystal formulation, wherein the nanocrystal is a tablet, comprising 22.20g of ROCK2 inhibitor, 10.00g of polysorbate 80, 20.00g of polyethylene glycol 6000, 44.80g of mannitol, 2.00g of silicon dioxide and 1.00g of sodium stearyl fumarate, alternatively, the particle size of the ROCK2 inhibitor is 50-1000nm, alternatively 50-500nm, yet alternatively 50-300nm, still alternatively 50-150nm.

In one embodiment, the present disclosure relates to a nanocrystal formulation, wherein the nanocrystal is a tablet, comprising 22.20g of ROCK2 inhibitor, 10.00g of polysorbate 80, 20.00g of poloxamer 188, 44.80g of mannitol, 2.00g of silicon dioxide and 1.00g of sodium stearyl fumarate, alternatively, the particle size of the ROCK2 inhibitor is 50-1000nm, alternatively 50-500nm, yet alternatively 50-300nm, still alternatively 50-150nm.

In one embodiment, the present disclosure relates to a nanocrystal formulation, wherein the nanocrystal is a tablet, comprising 22.20g of ROCK2 inhibitor, 10.00g of polysorbate 80, 20.00g of polyvinyl alcohol, 44.80g of mannitol, 2.00g of silicon dioxide and 1.00g of sodium stearyl fumarate, alternatively, the particle size of the ROCK2 inhibitor is 50-1000nm, alternatively 50-500nm, yet alternatively 50-300nm, still alternatively 50-150nm.

In one embodiment, the present disclosure relates to a nanocrystal formulation, wherein the nanocrystal is a tablet, comprising 22.20g of ROCK2 inhibitor, 10.00g of polysorbate 80, 16.00g of povidone K29/32, 4.00g of poloxamer 188, 44.80g of mannitol, 2.00g of silicon dioxide and 1.00g of sodium stearyl fumarate, alternatively, the particle size of the ROCK2 inhibitor is 50-1000nm, alternatively 50-500nm, yet alternatively 50-300nm, still alternatively 50-150nm.

In one embodiment, the present disclosure relates to a nanocrystal formulation, wherein the nanocrystal is a tablet, comprising 22.20g of ROCK2 inhibitor, 10.00g of polysorbate 80, 4.00g of povidone K29/32, 16.00g of poloxamer 188, 44.80g of mannitol, 2.00g of silicon dioxide and 1.00g of sodium stearyl fumarate, alternatively, the particle size of the ROCK2 inhibitor is 50-1000nm, alternatively 50-500nm, yet alternatively 50-300nm, still alternatively 50-150nm.

In one embodiment, the present disclosure relates to a nanocrystal formulation, wherein the nanocrystal is a tablet, comprising 22.20g of ROCK2 inhibitor, 10.00g of polysorbate 80, 10.00g of povidone K29/32, 10.00g of poloxamer 188, 44.80g of mannitol, 2.00g of silicon dioxide and 1.00g of sodium stearyl fumarate, alternatively, the particle size of the ROCK2 inhibitor is 50-1000nm, alternatively 50-500nm, yet alternatively 50-300nm, still alternatively 50-150nm.

In one embodiment, the present disclosure relates to a nanocrystal formulation, wherein the nanocrystal formulation is a capsule, comprising:
- 10-50% of ROCK2 inhibitor, alternatively 20-40% of ROCK2 inhibitor, yet alternatively 20%, 25%, 30%, 35% or 40% of ROCK2 inhibitor;
- 10-40% of stabilizer, alternatively 20-30% of stabilizer, yet alternatively 20%, 22%, 25%, 28% or 30% of stabilizer.

In one embodiment, the present disclosure relates to a nanocrystal formulation, wherein the nanocrystal is a capsule, comprising 22.20g of ROCK2 inhibitor, 6.00g of polysorbate 80, 8.00g of povidone K29/32, 4.00g of poloxamer and 20.00g of mannitol, alternatively, the particle size of the ROCK2 inhibitor is 50-1000nm, alternatively 50-500nm, yet alternatively 50-300nm, still alternatively 50-150nm.

In one embodiment, the present disclosure relates to a nanocrystal formulation, wherein the nanocrystal is a capsule, comprising 4.45g of ROCK2 inhibitor, 1.20g of polysorbate 80, 1.20g of povidone K32/29, 0.80g of poloxamer 188 and 6.80g of mannitol, alternatively, the particle size of the ROCK2 inhibitor is 50-1000nm, alternatively 50-500nm, yet alternatively 50-300nm, still alternatively 50-150nm.

In one embodiment, the present disclosure relates to a nanocrystal formulation, wherein the nanocrystal formulation is a nanocrystal enteric formulation selected from enteric-coated tablets and enteric capsules.

In one embodiment, the present disclosure relates to a nanocrystal formulation, wherein the nanocrystal formulation is a nanocrystal enteric-coated tablet, wherein the enteric coating material is selected from one or more of shellac, polyvinyl alcohol acetate phthalate (PVAP), methacrylic acid copolymer, cellulose and its derivatives (cellulose acetate phthalate (CAP), cellulose acetate trimellitate (CAT), hydroxypropyl cellulose phthalate (HPMCP)), and acrylic resins (EuS100, EuL100).

In one embodiment, the present disclosure relates to a nanocrystal formulation, wherein the nanocrystal formulation is a nanocrystal enteric capsule, wherein the enteric capsule is selected from gelatin enteric capsules and hypromellose enteric capsules, and the capsule material composition is selected from one or more of shellac, polyvinyl alcohol acetate phthalate (PVAP), methacrylic acid copolymer, cellulose and its derivatives (cellulose acetate phthalate (CAP), cellulose acetate trimellitate (CAT), hydroxypropyl cellulose phthalate (HPMCP)), and acrylic resin (EuS100, EuL100).

In one embodiment, the present disclosure relates to a nanocrystal formulation, wherein the nanocrystal formulation is a nanocrystal enteric-coated tablet, comprising:
- 1-20% of ROCK2 inhibitor, alternatively 5-15% of ROCK2 inhibitor, yet alternatively 5%, 6%, 7%, 8%, 9%, 10%, 11%, 12%, 13%, 14% or 15% of ROCK2 inhibitor; and
- 1-90% of stabilizer, alternatively 10-40% of stabilizer, yet alternatively 15-25% of stabilizer, for example, 15%, 16%, 17%, 18%, 19%, 20%, 21%, 22%, 23%, 24% or 25% of stabilizer.

In one embodiment, the present disclosure relates to a nanocrystal formulation, wherein the nanocrystal formulation is a nanocrystal enteric-coated tablet, comprising 5.56 g of ROCK2 inhibitor, 1.50 g of polysorbate 80, 1.00 g of poloxamer, 1.50 g of povidone K29/32, 5.00 g of mannitol, 1.00 g of silicon dioxide, 4.00 g of crospovidone, 29.94 g of microcrystalline cellulose, 0.50 g of magnesium stearate and 5 g of film coating premix (enteric type), alternatively, the particle size of the ROCK2 inhibitor is 50-1000nm, alternatively 50-500nm, yet alternatively 50-300nm, still alternatively 50-150nm.

In one embodiment, the present disclosure relates to a nanocrystal formulation, wherein the nanocrystal formulation is a nanocrystal enteric-coated tablet, comprising 5.56 g of ROCK2 inhibitor, 1.50 g of polysorbate 80, 1.00 g of poloxamer, 1.50 g of povidone K29/32, 38.94 g of mannitol, 1.00 g of silicon dioxide, 0.50 g of magnesium stearate and 5g of film coating premix (enteric type), alternatively, the particle size of the ROCK2 inhibitor is 50-1000nm, alternatively 50-500nm, yet alternatively 50-300nm, still alternatively 50-150nm.

In one embodiment, the present disclosure relates to a nanocrystal formulation, wherein the nanocrystal formulation is a nanocrystal enteric capsule, comprising:
- 5-30% of ROCK2 inhibitor, alternatively 10-20% of ROCK2 inhibitor, yet alternatively 10%, 11%, 12%, 13%, 14%, 15%, 16%, 17%, 18%, 19% or 20% of ROCK2 inhibitor; and
- 10-50% of stabilizer, alternatively 20-40% of stabilizer, yet alternatively 30-40% of stabilizer, for example, 30%, 31%, 32%, 33%, 34%, 35%, 36%, 37%, 38%, 39% or 40% of stabilizer.

In one embodiment, the present disclosure relates to a nanocrystal formulation, wherein the nanocrystal formulation is a nanocrystal enteric capsule, comprising 11.1 g of ROCK2 inhibitor, 3.0 g of polysorbate 80, 2.0 g of poloxamer, 3.0 g of povidone K29/32, 20.0 g of mannitol, 2.00 g of silicon dioxide, 8.0 g of crospovidone, 29.9 g of microcrystalline cellulose, 1.0 g of magnesium stearate and enteric capsules, alternatively, the particle size of the ROCK2 inhibitor is 50-1000nm, alternatively 50-500nm, yet alternatively 50-300nm, still alternatively 50-150nm.

In some embodiments of the present disclosure, in addition to the compound of formula (I), (II), (III) or formula (IV), or a pharmaceutically acceptable salt or hydrate thereof, the ROCK2 inhibitor may also optionally include at least one other compound having a synergistic therapeutic effect with the compound.

In one embodiment, the present disclosure relates to the preparation method of the above-mentioned nanocrystal formulation, which includes grinding a ROCK2 inhibitor and a stabilizer.

In one embodiment, the present disclosure relates to the preparation method of the above-mentioned nanocrystal formulation, wherein the weight ratio of the ROCK2 inhibitor to the stabilizer during grinding is 1:15 to 15:1, 1:14 to 14:1, 1:13 to 13:1, 1:12 to 12:1, 1:11 to 11:1, 1:10 to 10:1, 1:9 to 9:1, 1:8 to 8:1, 1:7 to 7:1, 1:6 to 6:1, 1:5 to 5:1, 1:4 to 4:1, 1:3 to 3:1, 1:2 to 2:1, 1:1; alternatively, the weight ratio of the ROCK2 inhibitor to the stabilizer during grinding is 15:1 to 2:1, yet alternatively 15:1, 14:1, 13:1, 12:1, 11:1, 10:1, 10:3, 9:1, 8:1,7:1,6:1,5:1,4:1,3:1 or 2:1.

In one embodiment, the present disclosure relates to the preparation method of the above-mentioned nanocrystal formulation, wherein the grinding medium is selected from porcelain balls, glass balls, zirconia beads, steel balls and ice beads; alternatively, the grinding medium is zirconia beads.

In one embodiment, the present disclosure relates to the preparation method of the above-mentioned nanocrystal formulation, wherein the particle size of the grinding medium ranges from 0.1 to 1 mm, alternatively from 0.1 to 0.5 mm, and yet alternatively 0.2 mm.

In one embodiment, the present disclosure relates to the preparation method of the above-mentioned nanocrystal formulation, wherein the grinding time is 0.1-6h, alternatively 0.5-6h, alternatively 4-6h, yet alternatively 10min, 20min, 30min, 40min, 1h, 1.5h, 2h, 2.5h, 3h, 3.5h, 4h, 4.5h, 5h, 5.5h or 6h.

In one embodiment, the present disclosure relates to the preparation method of the above-mentioned nanocrystal formulation, wherein the grinding speed is 1000~6000rpm, alternatively 1500rpm~4500rpm, yet alternatively 1500rpm, 2000rpm, 2500rpm, 3000rpm, 3500rpm, 4000rpm, 4500rpm, 5000rpm,5500rpm or 6000rpm.

In one embodiment, the present disclosure relates to the preparation method of the above-mentioned nanocrystal formulation, wherein the filling amount of the grinding beads is 50% to 95%, alternatively 70% to 90%, and yet alternatively 70%, 80% or 90%.

In one embodiment, the present disclosure relates to the preparation method of the above-mentioned nanocrystal formulation, which further includes a pre-grinding step before grinding the ROCK2 inhibitor and the stabilizer.

In one embodiment, the present disclosure relates to the preparation method of the above-mentioned nanocrystal formulation, wherein the pre-grinding speed is 3000~6000rpm, alternatively 3000rpm, 3500rpm, 4000rpm, 4500rpm, 5000rpm, 5500rpm or 6000rpm, yet alternatively 4000rpm; the pre-grinding time is 1-30 min, alternatively 2-20 min, alternatively 3 min, 4 min, 5 min, 6 min, 8 min, 10 min, 12 min, 15 min, 18 min or 20 min, yet alternatively 5 min.

In one embodiment, the present disclosure relates to the preparation method of the above-mentioned nanocrystal formulation, wherein a stabilizer and/or an excipient are optionally added after grinding.

In one embodiment, the present disclosure relates to the preparation method of the above-mentioned nanocrystal formulation, wherein the stabilizer added after grinding is selected from one or more of polysorbate, povidone, polyoxyethylene fatty acid ester, polyethylene glycol, polyvinyl alcohol, hydroxypropyl cellulose, hydroxypropyl methylcellulose, polyvinylpyrrolidone, poloxamer, sodium lauryl sulfate, sodium docusate, polyethylene glycol 15-hydroxystearate, polyoxyethylene castor oil, copovidone, lactose, and mannitol.

In one embodiment, the present disclosure relates to the preparation method of the above-mentioned nanocrystal formulation, wherein the stabilizer added after grinding is selected from one or more of povidone K29/32, poloxamer 188, polyvinyl alcohol, lactose and mannitol.

In one embodiment, the present disclosure relates to the preparation method of the above-mentioned nanocrystal formulation, wherein the stabilizer added after grinding is a mixture of povidone K29/32 and poloxamer 188, and the mixing ratio of the two is 1:10 to 10:1, alternatively 1:9 to 9:1, alternatively 1:8 to 8:1, alternatively 1:7 to 7:1, alternatively 1:6 to 6:1, alternatively 1:5 to 5:1, alternatively 1:4 to 4:1, alternatively 1:3 to 3:1, alternatively 1:2 to 2:1, alternatively 1:1; yet alternatively, the mixing ratio of the two is 1:4, 4:1 or 1:1.

In one embodiment, the present disclosure relates to the preparation method of the above-mentioned nanocrystal formulation, which includes:

the active ingredient, part of the stabilizer and purified water are ground with a nano grinder. After grinding, a nano suspension is obtained, and then stabilizers and/or fillers are added to obtain a nanocrystal suspension. Alternatively, after grinding to obtain a nanosuspension, spray drying or freeze drying is used to solidify to obtain a solid nanocrystal mixture; and then fillers are added and mixed, and lubricants are added; and then a shaping process is performed. In a specific implementation, the shaping process may include steps such as granulation, optional finishing, and tableting or capsule filling. The key process in the present disclosure is the nano-grinding process. When the average particle size of an active ingredient reaches 50nm~1000nm, further alternatively 80nm~500nm, yet alternatively 80nm~300nm, the grinding is completed. It should be noted that mixing and subsequent shaping processes are common processes and operations in the technical field, and the present disclosure is not specifically limited here.

In the process of preparing ROCK2 inhibitor nanocrystals, the inventor investigated the effects of grinding speed, filling amount of grinding bead, sample amount and grinding time on the particle size of the ROCK2 inhibitor. The results show that the particle size of the ROCK2 inhibitor gradually decreases with the increase of grinding time, but the decrease rate gradually becomes slower; as the grinding speed increases, the grinding efficiency increases; at the same rotation speed, the grinding efficiency increases as the filling amount of grinding beads increases. Sample volume has no significant effect on grinding efficiency. The research results of grinding parameters and particle size are shown in Table 8. Based on this, in some specific embodiments of the present disclosure, in the grinding process, 0.2 mm zirconia grinding beads are used, and the filling amount of the grinding beads is 50% to 95%, alternatively 70% to 90%. The grinding speed is 1000rpm~4500rpm, alternatively 1500rpm~3500rpm. The grinding time is 2h~6h, alternatively 4h~5h.

In some embodiments of the present disclosure, when the nanocrystal formulation composition comprises a lubricant, the ground nanosuspension needs to be spray-dried or freeze-dried to remove moisture, and then mixed with optional fillers directly or after crushing, and then
A: mixed with all lubricants, and then a shaping process is performed;
   or,
B: firstly, mixed with part of lubricants; then, granulating and optional finishing are performed; then, the remaining part of lubricants are added; and then, other shaping processes such as tableting or capsule filling are performed.

In one embodiment, the present disclosure relates to a method of preventing, alleviating and/or treating idiopathic pulmonary fibrosis, fatty liver disease and/or steatohepatitis, graft-versus-host disease after hematopoietic stem cell transplantion, or viral infections, wherein the method includes administering to a subject a therapeutically effective amount of the nanocrystal formulation or a nanocrystal formulation prepared by the method; alternatively, the method is a method for preventing, alleviating and/or treating fatty liver disease and/or steatohepatitis; alternatively, the fatty liver disease is alcoholic fatty liver disease (ALFD) or non-alcoholic fatty liver disease (NALFD), the steatohepatitis is alcoholic steatohepatitis (ASH) or non-alcoholic steatohepatitis (NASH), the hematopoietic stem cell transplantion is an allogeneic hematopoietic stem cell transplantion, the graft-versus-host disease is acute graft-versus-host disease or chronic graft-versus-host disease, and the viral infection is a coronavirus infection; alternatively, the coronavirus is selected from SARA-CoV, SARA-CoV-2, MERS-CoV, HCoV-229E, HCoV-NL63, HCoV-OC43 and HCoV-HKU1; alternatively, the disease caused by the coronavirus is Middle East Respiratory Syndrome, Severe Acute Respiratory Syndrome or COVID-19; alternatively, the coronavirus is Severe Acute Respiratory Syndrome coronavirus 2 (SARA-CoV-2 or 2019-nCoV), and the disease caused by the coronavirus is COVID-19.

In one embodiment, the present disclosure relates to the nanocrystal formulation or a nanocrystal formulation prepared by the method for use in preventing, alleviating and/or treating idiopathic pulmonary fibrosis, fatty liver disease and/or steatohepatitis, graft-versus-host disease after hematopoietic stem cell transplantion or viral infection; alternatively, the nanocrystal formulation is used for preventing, alleviating and/or treating fatty liver disease and/or steatohepatitis; alternatively, the fatty liver disease is alcoholic fatty liver disease (ALFD) or non-alcoholic fatty liver disease (NALFD), the steatohepatitis is alcoholic steatohepatitis (ASH) or non-alcoholic steatohepatitis (NASH), the hematopoietic stem cell transplantion is an allogeneic hematopoietic stem cell transplantion, the graft-versus-host disease is acute graft-versus-host disease or chronic graft-versus-host disease, and the viral infection is a coronavirus infection; alternatively, the coronavirus is selected from SARA-CoV, SARA-CoV-2, MERS-CoV, HCoV-229E, HCoV-NL63, HCoV-OC43 and HCoV-HKU1; alternatively, the disease caused by the coronavirus is Middle East Respiratory Syndrome, Severe Acute Respiratory Syndrome or COVID-19; alternatively, the coronavirus is Severe Acute Respiratory Syndrome coronavirus 2 (SARA-CoV-2 or 2019-nCoV), and the disease caused by the coronavirus is COVID-19.

In one embodiment, the present disclosure relates to use of the nanocrystal formulation or a nanocrystal formulation prepared by the method in the manufacture of a medicament for preventing, alleviating and/or treating idiopathic pulmonary fibrosis, fatty liver disease and/or steatohepatitis, graft-versus-host disease after hematopoietic stem cell transplantion, or viral infections; alternatively, the use is a use in the manufacture of a medicament for preventing, alleviating, and/or treating fatty liver disease and/or steatohepatitis; alternatively, the fatty liver disease is alcoholic fatty liver disease (ALFD) or non-alcoholic fatty liver disease (NALFD), the steatohepatitis is alcoholic steatohepatitis (ASH) or non-alcoholic steatohepatitis (NASH), the hematopoietic stem cell transplantion is an allogeneic hematopoietic stem cell transplantion, the graft-versus-host disease is acute graft-versus-host disease or chronic graft-versus-host disease, and the viral infection is a coronavirus infection; alternatively, the coronavirus is selected from SARA-CoV, SARA-CoV-2, MERS-CoV, HCoV-229E, HCoV-NL63, HCoV-OC43 and HCoV-HKU1; alternatively, the disease caused by the coronavirus is Middle East Respiratory Syndrome, Severe Acute Respiratory Syndrome or COVID-19; alternatively, the coronavirus is Severe Acute Respiratory Syndrome coronavirus 2 (SARA-CoV-2 or 2019-nCoV), and the disease caused by the coronavirus is COVID-19.

In order to make the solved technical problems, technical solutions and beneficial effects of the present disclosure clearer, the present disclosure will be further described below in conjunction with specific examples. In the following examples, unless otherwise stated, the specific conditions of the test methods are usually implemented according to conventional conditions or conditions recommended by a manufacturer; the raw materials and reagents are all commercially available or prepared using public information.

### Example

The active pharmaceutical ingredient (API) used in the following comparative examples, examples and tests are all the aforementioned compound of formula (IV).

### I. Preparation of comparative examples

### Comparative example 1

### Formula:

| Drugs/Excipients | Proportion (%) | Dosage (mg) |
|---|---|---|
| API hydrochloride | 33.3 | 199.80 |
| Lactose | 28.6 | 171.60 |
| Microcrystalline cellulose | 34.5 | 207.00 |
| Croscarmellose sodium | 3.04 | 18.24 |
| Magnesium stearate | 0.57 | 3.42 |
| Tablet weight | 100 | 600.00mg |

Preparation method: The amounts of API hydrochloride, lactose, microcrystalline cellulose and croscarmellose sodium in the formula were weighed, mixed, and dry-granulated. After finishing, magnesium stearate was added, mixed, and tableted.

### Comparative example 2

### Formula:

| Drugs/Excipients | Proportion (%) | Dosage (mg) |
|---|---|---|
| API hydrochloride | 33.3 | 199.80 |
| Lactose | 28.6 | 171.60 |
| Microcrystalline cellulose | 34.5 | 207.00 |
| Croscarmellose sodium | 3.04 | 18.24 |
| Magnesium stearate | 0.57 | 3.42 |
| Tablet weight | 100 | 600.00mg |

Preparation method: First, the API hydrochloride was micronized to obtain API hydrochloride with a D₉₀ particle size of about 2 µm. The amounts of micronized API hydrochloride, lactose, microcrystalline cellulose, and croscarmellose sodium in the formula were weighed, mixed, and dry-granulated. After finishing, magnesium stearate was added, mixed, and tableted.

### Comparative example 3

### Formula:

| Drugs/Excipients | Proportion (%) | Dosage (mg) |
|---|---|---|
| API hydrochloride | 37.1 | 222.60 |
| Silicon dioxide | 5 | 30.00 |
| Pregelatinized starch 1500 | 20 | 120.00 |
| Microcrystalline cellulose | 28.9 | 173.40 |
| Crospovidone | 8 | 48.00 |
| Magnesium stearate | 1 | 6.00 |
| Tablet weight | 100 | 600.00mg |

Preparation method: The amounts of API hydrochloride, silica, pregelatinized starch 1500, and microcrystalline cellulose and half the amounts of crospovidone and magnesium stearate in the formula were weighed, mixed, and dry-granulated. After finishing, the other half of crospovidone and magnesium stearate were added, mixed, and tableted.

### II. Preparation of nanocrystal formulation (Suspension)

### Example 1

### Formula:

| Drugs/Excipients | Proportion (%) | Dosage (g) |
|---|---|---|
| API hydrochloride | 4.5 | 11.13 |
| Polysorbate 80 | 0.3 | 0.77 |
| Povidone K29/32 | 2.0 | 5.00 |
| Purified water | 93.2 | 233.10 |
| Production | | 250.00g |

Preparation method: The amounts of API hydrochloride, polysorbate 80 and part of purified water in the formula were weighed. The mixture was ground with a nano-grinder (0.2mm grinding beads, 70% filling amount) with grinding speed of 1500rpm, grinding time of 10min, to obtain a nano-suspension with an average particle size of 1868nm. A solution of povidone K29/32 was added to the nano-suspension and the mixture was diluted to 250g to obtain a final product with a concentration of 40mg/mL (based on free base) and a particle size of 1875nm.

### Example 2

Referring to the formula of Example 1, the grinding speed was 1500rpm and the time was 20min to obtain a nanosuspension with an average particle size of 1006nm. Then a solution of povidone K29/32 was added to the nanosuspension and the mixture was diluted to 250g to obtain a final product with a concentration of 40mg/mL (based on free base) and a particle size of 1080nm.

### Example 3

Referring to the formula of Example 1, the grinding speed was 1500rpm and the time was 40min to obtain a nanosuspension with an average particle size of 512nm. Then a solution of povidone K29/32 was added to the nanosuspension and the mixture was diluted to 250g to obtain a final product with a concentration of 40mg/mL (based on free base) and a particle size of 540nm.

### Example 4

### Formula:

| Drugs/Excipients | Proportion (%) | Dosage (g) |
|---|---|---|
| API hydrochloride | 4.5 | 11.13 |
| Polysorbate 80 | 0.3 | 0.77 |
| Povidone K29/32 | 1.0 | 2.50 |
| Purified water | 94.2 | 235.60 |
| Production | | 250.00g |

Preparation method: The amounts of API hydrochloride, polysorbate 80 and part of purified water in the formula were weighed. The mixture was ground with a nano-grinder (0.2mm grinding beads, 70% filling amount) with grinding speed of 1500rpm, grinding time of 6h, to obtain a nano-suspension with an average particle size of 268nm. A solution of povidone K29/32 was added to the nano-suspension and the mixture was diluted to 250g to obtain a final product with a concentration of 40mg/mL (based on free base) and a particle size of 300nm.

### Example 5

### Formula:

| Drugs/Excipients | Proportion (%) | Dosage (g) |
|---|---|---|
| API hydrochloride | 4.5 | 11.13 |
| Polysorbate 80 | 0.3 | 0.77 |
| Povidone K29/32 | 2.0 | 5.00 |
| Purified water | 93.2 | 233.10 |
| Production | | 250.00g |

Preparation method: The amounts of API hydrochloride, polysorbate 80 and part of purified water in the formula were weighed. The mixture was ground with a nano-grinder (0.2mm grinding beads, 70% filling amount) with grinding speed of 1500rpm, grinding time of 6h, to obtain a nanosuspension with an average particle size of 268nm. A solution of povidone K29/32 was added to the nano-suspension and the mixture was diluted to 250g to obtain a final product with a concentration of 40mg/mL (based on free base) and a particle size of 302nm.

### Example 6

### Formula:

| Drugs/Excipients | Proportion (%) | Dosage (g) |
|---|---|---|
| API hydrochloride | 4.5 | 11.13 |
| Polysorbate 80 | 0.3 | 0.77 |
| Hypromellose | 1.0 | 2.50 |
| Purified water | 94.2 | 235.60 |
| Production | | 250.00g |

Preparation method: The amounts of API hydrochloride, polysorbate 80 and part of purified water in the formula were weighed. The mixture was ground with a nano-grinder (0.2mm grinding beads, 70% filling amount) with grinding speed of 1500rpm, grinding time of 6h, to obtain a nanosuspension with an average particle size of 268nm. A solution of hypromellose was added to the nano-suspension and the mixture was diluted to 250g to obtain a final product with a concentration of 40mg/mL (based on free base) and a particle size of 336nm.

### Example 7

### Formula:

| Drugs/Excipients | Proportion (%) | Dosage (g) |
|---|---|---|
| API hydrochloride | 4.5 | 11.13 |
| Polysorbate 80 | 0.3 | 0.77 |
| Hypromellose | 2.0 | 5.00 |
| Purified water | 93.2 | 233.10 |
| Production | | 250.00g |

Preparation method: The amounts of API hydrochloride, polysorbate 80 and part of purified water in the formula were weighed. The mixture was ground with a nano-grinder (0.2mm grinding beads, 70% filling amount) with grinding speed of 1500rpm, grinding time of 6h, to obtain a nanosuspension with an average particle size of 268nm. A solution of hypromellose was added to the nano-suspension and the mixture was diluted to 250g to obtain a final product with a concentration of 40mg/mL (based on free base) and a particle size of 442nm.

### Example 8

### Formula:

| Drugs/Excipients | Proportion (%) | Dosage (g) |
|---|---|---|
| API hydrochloride | 4.48 | 250.08 |
| Polysorbate 80 | 0.31 | 17.40 |
| Povidone K29/32 | 2.00 | 111.60 |
| Methylparaben | 0.18 | 10.00 |
| Propylparaben | 0.02 | 1.10 |
| Purified water | 93.01 | 5189.82 |
| Production | | 5580.00g |

Preparation method: The amounts of API hydrochloride, polysorbate 80 and part of purified water in the formula were weighed. The mixture was ground with a nano-grinder (0.2mm grinding beads, 70% filling amount) with grinding speed of 1500rpm, grinding time of 4h, to obtain a nanosuspension with an average particle size of 281nm. A pre-prepared solution of methylparaben, propylparaben and povidone K29/32 was added to the nano-suspension. The mixture was diluted to 5580g to obtain a final product with a concentration of 40mg/mL (based on free base) and a particle size of 283nm.

### Example 9

### Formula:

| Drugs/Excipients | Proportion (%) | Dosage (g) |
|---|---|---|
| API hydrochloride | 4.00 | 55.19 |
| Polysorbate 80 | 1.50 | 18.61 |
| Polyoxyethylene castor oil | 8.00 | 99.25 |
| Methylparaben | 0.18 | 2.23 |
| Propylparaben | 0.02 | 0.25 |
| Purified water | 85.85 | 1065.09 |
| Production | | 1240.62g |

Preparation method: The amounts of API hydrochloride, polysorbate 80 and part of purified water in the formula were weighed. The mixture was ground with a nano-grinder (0.2mm grinding beads, 90% filling amount) with grinding speed of 3000rpm, grinding time of 4h, to obtain a nanosuspension with an average particle size of 100nm. A pre-prepared solution of methylparaben, propylparaben and polyoxyethylene castor oil was added to the nano-suspension. The mixture was diluted to 1240.62g to obtain a final product with a concentration of 40mg/mL (based on free base) and a particle size of 106nm.

### III. Preparation of nanocrystal formulation (Tablets)

### Example 10

### Formula:

| Drugs/Excipients | Proportion (%) | Dosage (g) |
|---|---|---|
| API hydrochloride | 25.8 | 11.70 |
| Polysorbate 80 | 7.0 | 3.15 |
| Lactose | 11.6 | 5.26 |
| Polyethylene glycol 6000 | 2.3 | 1.05 |
| Mannitol | 35.4 | 16.03 |
| Silicon dioxide | 10.9 | 4.94 |
| Sodium dodecyl sulfate | 5.4 | 2.47 |
| Microcrystalline cellulose | 6.5 | 2.96 |
| Croscarmellose sodium | 6.5 | 2.96 |
| Magnesium stearate | 0.7 | 0.32 |
| Production | | 50.84g |

Preparation method: The amounts of API hydrochloride, part of polysorbate 80 (API free base:polysorbate 80=1:0.1) and part of purified water in the formula were weighed. The mixture was ground with a nano-grinder (0.2mm grinding beads, 90% filling amount) with grinding speed of 3000rpm, grinding time of 4h, to obtain a nanosuspension with an average particle size of 105nm. A pre-prepared solution of polysorbate 80, polyethylene glycol 6000 and lactose was added to the nano-suspension (API free base:polysorbate 80:lactose:polyethylene glycol 6000=1:0.3:0.5:0.1). The mixture was spray-dried with an air inlet temperature of 120 °C and a spray speed of 40rpm, to obtain a spray-dried powder. The mannitol, silicon dioxide, sodium lauryl sulfate, microcrystalline cellulose, croscarmellose sodium and magnesium stearate were added to an appropriate amount of the above spray-dried powder, mixed, and directly pressed into tablets. The tablet weight was about 1g (specification: 200mg, based on anhydrous API).

### Example 11

### Formula:

| Drugs/Excipients | Proportion (%) | Dosage (g) |
|---|---|---|
| API hydrochloride | 20.4 | 32.71 |
| Polysorbate 80 | 5.5 | 8.82 |
| Lactose | 9.2 | 14.71 |
| Polyethylene glycol 6000 | 1.8 | 2.94 |
| Mannitol | 27.3 | 43.63 |
| Silicon dioxide | 10.0 | 16.00 |
| Sodium dodecyl sulfate | 5.0 | 8.00 |
| Microcrystalline cellulose | 8.0 | 12.80 |
| Croscarmellose sodium | 8.0 | 12.80 |
| Sodium stearyl fumarate | 1.0 | 1.60 |
| Production | | 154.02g |

Preparation method: The amounts of API hydrochloride, part of polysorbate 80 (API free base:polysorbate 80=1:0.1) and part of purified water in the formula were weighed. The mixture was ground with a nano-grinder (0.2mm grinding beads, 90% filling amount) with grinding speed of 3000rpm, grinding time of 4h, to obtain a nanosuspension with an average particle size of 105nm. A pre-prepared solution of polysorbate 80, polyethylene glycol 6000, part of the mannitol and lactose was added to the nano-suspension (API free base:polysorbate 80:lactose:mannitol:polyethylene glycol 6000=1:0.3:0.5:0.3:0.1). The mixture was spray-dried with an air inlet temperature of 120 °C, spray speed of 40 rpm, to obtain a spray dried powder. The silicon dioxide was added to an appropriate amount of the above spray-dried powder, mixed, and passed through a 35-mesh sieve. Then sodium lauryl sulfate, mannitol, microcrystalline cellulose, and croscarmellose sodium (intra) were added to the powder, and mixed. The mixture was wet-granulated with an aqueous solution of 10% sodium lauryl sulfate, dried, and finished. The granules were then mixed with croscarmellose sodium (extra) and sodium stearyl fumarate, and pressed into tablets. The tablet weight was about 1g (specification: 200mg, based on anhydrous API).

### Example 12

### Formula:

| Drugs /Excipients | Proportion (%) | Dosage (g) |
|---|---|---|
| API hydrochloride | 22.3 | 3.34 |
| Polysorbate 80 | 5.9 | 0.89 |
| Lactose | 12.0 | 1.80 |
| Polyethylene glycol 6000 | 2.0 | 0.30 |
| Silicon dioxide | 5.0 | 0.75 |
| Sodium dodecyl sulfate | 5.0 | 0.75 |
| Microcrystalline cellulose | 38.8 | 5.82 |
| Croscarmellose sodium | 8.0 | 1.20 |
| Sodium stearyl fumarate | 1.0 | 0.15 |
| Production | | 15.00g |

Preparation method: The amounts of API hydrochloride, polysorbate 80 and part of purified water in the formula were weighed. The mixture was ground with a nano-grinder (0.2mm grinding beads, 90% filling amount) with grinding speed of 4500rpm, grinding time of 6h, to obtain nanocrystal suspension with a particle size of 58nm. A pre-prepared solution of polyethylene glycol 6000 and lactose was added to the nanocrystal suspension, and the mixture was spray-dried. The air inlet temperature was 120 °C and the spray speed was 40 rpm to obtain spray-dried powder. The silicon dioxide was added to an appropriate amount of the above spray-dried powder, mixed, and passed through a 35-mesh sieve. Then microcrystalline cellulose, croscarmellose sodium (intra), and sodium stearyl fumarate (intra) were added to the powder, mixed, dry granulated, and finished through a 24-mesh sieve. Then croscarmellose sodium (extra) and sodium stearyl fumarate (extra) were added to the granules, mixed, and pressed into tablets. The tablet weight was about 1g (specification: 200mg, based on anhydrous API).

### Example 13

### Formula:

| Drugs/Excipients | Proportion (%) | Dosage (g) |
|---|---|---|
| API hydrochloride | 22.3 | 3.34 |
| Polysorbate 80 | 5.9 | 0.89 |
| Lactose | 12.0 | 1.80 |
| Polyethylene glycol 6000 | 2.0 | 0.30 |
| Silicon dioxide | 5.0 | 0.75 |
| Sodium dodecyl sulfate | 3.0 | 0.45 |
| Microcrystalline cellulose | 30.0 | 4.50 |
| Pregelatinized starch | 10.8 | 1.62 |
| Croscarmellose sodium | 8.0 | 1.20 |
| Sodium stearyl fumarate | 1.0 | 0.15 |
| Production | | 15.00g |

Preparation method: The amounts of API hydrochloride, polysorbate 80 and part of purified water in the formula were weighed. The mixture was ground with a nano-grinder (0.2mm grinding beads, 90% filling amount) with grinding speed of 4500rpm, grinding time of 6h, to obtain nanocrystal suspension with a particle size of 58nm. A pre-prepared solution of polyethylene glycol 6000 and lactose was added to the nanocrystal suspension, and the mixture was spray-dried. The air inlet temperature was 120 °C and the spray speed was 40 rpm to obtain spray-dried powder. The silicon dioxide was added to an appropriate amount of the above spray-dried powder, mixed, and passed through a 35-mesh sieve. Then microcrystalline cellulose, pregelatinized starch, croscarmellose sodium (intra), and sodium stearyl fumarate (intra) were added to the powder, dry granulated, and finished through a 24-mesh sieve. Then croscarmellose sodium (extra) and sodium stearyl fumarate (extra) were added to the granules, mixed, and pressed into tablets. The tablet weight was about 1g (specification: 200mg, based on anhydrous API).

### Example 14

### Formula:

| Drugs/Excipients | Proportion (%) | Dosage (g) |
|---|---|---|
| API hydrochloride | 22.3 | 11.14 |
| Polysorbate 80 | 5.9 | 2.97 |
| Lactose | 12.0 | 6.01 |
| Polyethylene glycol 6000 | 2.0 | 1.00 |
| Silicon dioxide | 2.0 | 1.00 |
| Spray-dried mannitol | 54.8 | 27.38 |
| Sodium stearyl fumarate | 1.0 | 0.50 |
| Production | | 50.00g |

Preparation method: The amounts of API hydrochloride, polysorbate 80 and part of purified water in the formula were weighed. The mixture was ground with a nano-grinder (0.2mm grinding beads, 90% filling amount) with grinding speed of 3000rpm, grinding time of 6h, to obtain nanocrystal suspension with a particle size of 89nm. A pre-prepared solution of polyethylene glycol 6000 and lactose was added to the nanocrystal suspension, and the mixture was spray-dried. The air inlet temperature was 120 °C and the spray speed was 40 rpm to obtain a spray-dried powder. The silicon dioxide, spray-dried mannitol and sodium stearyl fumarate were added to an appropriate amount of the above spray-dried powder, mixed, and pressed into tablets. The tablet weight was about 1g (specification: 200mg, based on anhydrous API).

### Example 15

### Formula:

| Drugs/Excipients | Proportion (%) | Dosage (g) |
|---|---|---|
| API hydrochloride | 22.2 | 111.20 |
| Polysorbate 80 | 6.0 | 30.04 |
| Lactose | 12.0 | 59.99 |
| Polyethylene glycol 6000 | 2.0 | 10.00 |
| Silicon dioxide | 2.0 | 10.00 |
| Spray-dried mannitol | 54.8 | 273.78 |
| Sodium stearyl fumarate | 1.0 | 5.00 |
| Production | | 500.09g |

Preparation method: The amounts of API hydrochloride, polysorbate 80 and part of purified water in the formula were weighed. The mixture was ground with a nano-grinder (0.2mm grinding beads, 90% filling amount) with grinding speed of 3000rpm, grinding time of 4h, to obtain nanocrystal suspension with a particle size of 128nm. A pre-prepared solution of polyethylene glycol 6000 and lactose was added to the nano-suspension. The mixture was freeze-dried (the solid content of the sample solution was about 20%), that is, pre-frozen at -40 °C for 5 hours and mainly dried at -5 °C for 13 hours with a vacuum degree of 0.18mbar, and secondarily dried at 10-15 °C for 16 hours with a vacuum degree of 0.18mbar. After the freeze-drying was completed, the sample was ground and finished using a finishing grinding mill. The silicon dioxide, spray-dried mannitol and sodium stearyl fumarate (intra) were added to the sample and mixed. The mixture was dry-granulated, and sodium stearyl fumarate was added (extra), mixed and pressed into tablets. The tablet weight was about 1g (specification: 200mg, based on anhydrous API).

### Example 16

### Formula:

| Drugs/Excipients | Proportion (%) | Dosage (g) |
|---|---|---|
| API hydrochloride | 22.2 | 22.22 |
| Polysorbate 80 | 20.0 | 20.0 |
| Mannitol | 54.8 | 54.8 |
| Silicon dioxide | 2.0 | 2.0 |
| Sodium stearyl fumarate | 1.0 | 1.0 |
| Production | | 100.02g |

Preparation method: The amounts of API hydrochloride, part of polysorbate 80 and part of purified water (API free base:polysorbate 80 = 1:0.3) in the formula were weighed. The mixture was ground with a nano-grinder (0.2mm grinding beads, 90% filling amount) with a grinding speed of 3000rpm, grinding time of 4.5h, to obtain a nanocrystal suspension with a particle size of 102nm. Then a pre-prepared solution of polysorbate 80 and mannitol was added to the nanocrystal suspension. The mixture was freeze-dried (the solid content of the sample solution was about 10%), that is, pre-frozen at -40 °C for 5 hours and mainly dried at -5 °C for 13 hours with a vacuum degree of 0.18mbar, and secondarily dried at 10-15 °C for 16 hours with a vacuum degree of 0.18mbar. After the freeze-drying was completed, the sample was ground and finished using a finishing grinding mill. The silicon dioxide, spray-dried mannitol and sodium stearyl fumarate were added to the sample, mixed, and pressed into tablets. The tablet weight was about 1g (specification: 200 mg, based on anhydrous API).

### Examples 17-23

Referring to the preparation method of Example 16, the grinding formula was API free base:polysorbate 80 = 1:0.3. After grinding, the stabilizer in the following table was added to the grinding liquid and dispersed evenly, and the mixture was freeze-dried. After drying, according to the formula in the table below, fillers, lubricants, etc. were added to achieve Examples 17-23. The particle sizes of each example after grinding were recorded in Table 7.

| Example | 16 | 17 | 18 | 19 | 20 | 21 | 22 | 23 |
|---|---|---|---|---|---|---|---|---|
| Ingredient name | Formula proportio n (%) | Formula proportio n (%) | Formula proportio n (%) | Formula proportio n (%) | Formula proportio n (%) | Formula proportio n (%) | Formula proportio n (%) | Formula proportio n (%) |
| API hydrochloride | 22.2 | 22.2 | 22.2 | 22.2 | 22.2 | 22.2 | 22.2 | 22.2 |
| Polysorbate 80 | 20.0 | 10 | 10 | 10 | 10 | 10 | 10 | 10 |
| Povidone K29/32 | 0 | 20 | 0 | 0 | 0 | 16 | 4 | 10 |
| Polyethylene glycol 6000 | 0 | 0 | 20 | 0 | 0 | 0 | 0 | 0 |
| Poloxamer 188 | 0 | 0 | 0 | 20 | 0 | 4 | 16 | 10 |
| Polyvinyl alcohol | 0 | 0 | 0 | 0 | 20 | 0 | 0 | 0 |
| Mannitol | 54.8 | 44.8 | 44.8 | 44.8 | 44.8 | 44.8 | 44.8 | 44.8 |
| Silicon dioxide | 2.0 | 2.0 | 2.0 | 2.0 | 2.0 | 2.0 | 2.0 | 2.0 |
| Sodium stearyl fumarate | 1.0 | 1.0 | 1.0 | 1.0 | 1.0 | 1.0 | 1.0 | 1.0 |
| Tablet weight | 1000 | 1000 | 1000 | 1000 | 1000 | 1000 | 1000 | 1000 |

### IV. Preparation of nanocrystal formulations (Capsules)

### Example 24

### Formula:

| Drugs/Excipients | Proportion (%) | Dosage (g) |
|---|---|---|
| API hydrochloride | 37.0 | 22.20 |
| Polysorbate 80 | 10.0 | 6.00 |
| Povidone K29/32 | 13.3 | 8.00 |
| Poloxamer 188 | 6.6 | 4.00 |
| Mannitol | 33.2 | 20.00 |
| Production | | 60.20g |

Preparation method: The amounts of API hydrochloride, polysorbate 80 and purified water (API free base:polysorbate 80=1:0.3) in the formula were weighed. The mixture was ground with a nano-grinder (0.2mm grinding beads, 90% filling amount) with a grinding speed of 3000rpm and a grinding time of 5h, to obtain a nanocrystal suspension with an average particle size of 93nm. Then a pre-prepared solution of povidone K29/32, poloxamer 188 and mannitol was added to the nanocrystal suspension to afford API free base:polysorbate 80:povidone K29/32:poloxamer 188:mannitol = 1:0.3:0.4:0.2:1 in the final sample. The sample was freeze-dried (the solid content of the sample solution was about 10%), that is, pre-frozen at -40 °C for 5 hours and mainly dried at -5 °C for 13 hours with a vacuum degree of 0.18mbar, and secondarily dried at 10-15 °C for 16 hours with a vacuum degree of 0.18mbar. After the freeze-drying was completed, the sample was passed through a 40-mesh sieve, and 301.2 mg of the freeze-dried powder was weighed and directly filled into capsules, with a specification of 100 mg.

### Example 25

### Formula:

| Drugs/Excipients | Proportion (%) | Dosage (g) |
|---|---|---|
| API hydrochloride | 30.9 | 4.45 |
| Polysorbate 80 | 8.3 | 1.20 |
| Povidone K29/32 | 8.3 | 1.20 |
| Poloxamer 188 | 5.6 | 0.80 |
| Mannitol | 47.2 | 6.8 |
| Production | | 14.4g |

Preparation method: The amounts of API hydrochloride, polysorbate 80 and purified water (API free base:polysorbate 80=1:0.3) in the formula were weighed. The mixture was ground with a nano-grinder (0.2mm grinding beads, 90% filling amount) with a grinding speed of 3000rpm, grinding time of 5h, to obtain the nanocrystal suspension with an average particle size of 103nm. Then a pre-prepared solution of povidone K29/32, poloxamer 188 and mannitol was added to the nanocrystal suspension to afford API free base:polysorbate 80:povidone K29/32:poloxamer 188:mannitol = 1:0.3:0.3:0.2:1.7 in the final sample. The sample was freeze-dried (the solid content of the sample solution was about 10%), that is, pre-frozen at -40 °C for 5 hours and mainly dried at -5 °C for 13 hours with a vacuum degree of 0.18mbar, and secondarily dried at 10-15 °C for 16 hours with a vacuum degree of 0.18mbar. After the freeze-drying was completed, the sample was passed through a 40-mesh sieve, and 361 mg of the freeze-dried powder was weighed and directly filled into capsules, with a specification of 100 mg.

### V. Preparation of enteric nanocrystal formulations (tablets or capsules)

### Example 26 Enteric nanocrystal tablets

### Formula:

| Drugs/Excipients | Proportion (%) | Dosage (g) |
|---|---|---|
| API hydrochloride | 11.1 | 5.56 |
| Polysorbate 80 | 3.0 | 1.50 |
| Poloxamer | 2.0 | 1.00 |
| Povidone K29/32 | 3.0 | 1.50 |
| Mannitol | 10.0 | 5.00 |
| Silicon dioxide | 2.0 | 1.00 |
| Crospovidone | 8.0 | 4.00 |
| Microcrystalline cellulose | 59.9 | 29.94 |
| Magnesium stearate | 1.0 | 0.50 |
| Film coating premix (enteric type) | 9.9 | 5 |
| Production | | 55g |

Preparation method: The amounts of API hydrochloride, polysorbate 80 and part of purified water in the formula were weighed. The mixture was ground with a nano-grinder (0.2mm grinding beads, 90% filling amount) with grinding speed of 3000rpm, grinding time of 6h, to obtain nanocrystal suspension with a particle size of 109nm. Then a pre-prepared solution of poloxamer, povidone K29/32, and mannitol was added into the nanocrystal suspension. The mixture was freeze-dried (the solid content of the sample solution was about 20%), that is, pre-frozen at -40 °C for 5 hours and mainly dried at -5 °C for 13 hours with a vacuum degree of 0.18mbar, and secondarily dried at 10-15 °C for 16 hours with a vacuum degree of 0.18mbar. After the freeze-drying was completed, the sample was ground and finished using a finishing grinding mill. The silicon dioxide, microcrystalline cellulose, crospovidone and magnesium stearate were added to the sample, mixed, and pressed into tablets. The tablet weight was about 1g (specification: 100mg, based on anhydrous API). Film coating premix (enteric type) was used for enteric coating to prepare coating materials with a solid content of 20%, and the coating was performed under stirring. The air inlet temperature was 45°C, the air volume was 200m³/min, the tablet bed temperature was 31°C, the coating pot rotation speed was 15-18 rpm, the coating liquid spray speed was 5g/min, and the coating weight gain was 9.9%.

### Example 27 Enteric nanocrystal tablets

### Formula:

| Drugs/Excipients | Proportion (%) | Dosage (g) |
|---|---|---|
| API hydrochloride | 11.1 | 5.56 |
| Polysorbate 80 | 3.0 | 1.50 |
| Poloxamer | 2.0 | 1.00 |
| Povidone K29/32 | 3.0 | 1.50 |
| Mannitol | 77.9 | 38.94 |
| Silicon dioxide | 2.0 | 1.00 |
| Magnesium stearate | 1.0 | 0.50 |
| Film coating premix (enteric type) | 10.0 | 5 |
| Production | | 55g |

Preparation method: The amounts of API hydrochloride, polysorbate 80 and part of purified water in the formula were weighed. The mixture was ground with a nano-grinder (0.2mm grinding beads, 90% filling amount) with grinding speed of 3000rpm, grinding time of 6h, to obtain nanocrystal suspension with a particle size of 109nm. Then a pre-prepared solution of poloxamer, povidone K29/32, and mannitol (mannitol accounted for 15%) was added to the nanocrystal suspension. The mixture was freeze-dried (the solid content of the sample solution was about 20%), that is, pre-frozen at -40 °C for 5 hours and mainly dried at -5 °C for 13 hours with a vacuum degree of 0.18mbar, and secondarily dried at 10-15 °C for 16 hours with a vacuum degree of 0.18mbar. After the freeze-drying was completed, the sample was ground and finished using a finishing grinding mill. The silicon dioxide, mannitol (33.94g) and magnesium stearate were added to the sample, mixed, and pressed into tablets. The tablet weight was about 1g (specification: 100mg, based on anhydrous API). Film coating premix (enteric type) was used for enteric coating to prepare coating materials with a solid content of 20%, and the coating was performed under stirring. The air inlet temperature was 48°C, the air volume was 200m³/min, the tablet bed temperature was 36°C, the coating pot rotation speed was 15~20 rpm, the coating liquid spray speed was 5g/min, and the coating weight gain was 10.0%.

### Example 28 Enteric nanocrystal capsules

### Formula:

| Drugs/Excipients | Proportion (%) | Dosage (g) |
|---|---|---|
| API hydrochloride | 13.9 | 11.1 |
| Polysorbate 80 | 3.7 | 3.0 |
| Poloxamer | 2.5 | 2.0 |
| Povidone K29/32 | 3.7 | 3.0 |
| Mannitol | 25.0 | 20.0 |
| Silicon dioxide | 2.5 | 2.0 |
| Crospovidone | 10.0 | 8.0 |
| Microcrystalline cellulose | 37.4 | 29.9 |
| Magnesium stearate | 1.3 | 1.0 |
| Enteric capsules | - | |
| Production | | 80g |

Preparation method: The amounts of API hydrochloride, polysorbate 80 and part of purified water in the formula were weighed. The mixture was ground with a nano-grinder (0.2mm grinding beads, 90% filling amount) with grinding speed of 3000rpm, grinding time of 6h, to obtain nanocrystal suspension with a particle size of 109nm. Then a pre-prepared solution of poloxamer, povidone K29/32, and mannitol (mannitol accounted for 15%) was added to the nanocrystal suspension. The mixture was freeze-dried (the solid content of the sample solution was about 20%), that is, pre-frozen at -40 °C for 5 hours and mainly dried at -5 °C for 13 hours with a vacuum degree of 0.18mbar, and secondarily dried at 10-15 °C for 16 hours with a vacuum degree of 0.18mbar. After the freeze-drying was completed, the sample was ground and finished using a finishing grinding mill. The silicon dioxide, microcrystalline cellulose, mannitol (15g), crospovidone and magnesium stearate (0.25g) were added to the sample, mixed, dry-granulated, and finished through a 24-mesh sieve. Magnesium stearate (0.25g) was added and mixed. The sample was manually filled into enteric capsules, No. 00, with a filling amount of 800mg (specification 100mg, based on anhydrous API).

### Drug dissolution testing

### Test I

### Tablet dissolution test of Comparative Examples 1-3

### 1. Dissolution method:

1) Method: Dissolution determination method (Chinese Pharmacopoeia 2020 Edition, Part 4 General Chapter "0931 Dissolution and Release Determination Method" Second Method (Paddle Method));
2) Dissolution medium: purified water, 0.3 SDS aqueous solution, 0.5% SDS aqueous solution, 0.8% SDS aqueous solution, 1.0% SDS aqueous solution, pH 2.0 hydrochloric acid solution + 1.0% SDS, pH 4.5 acetate solution + 1.0% SDS, pH 6.8 phosphate solution + 1.0% SDS, 3% Tween solution; 900ml;
3) Speed: 75 rpm;
4) Sampling time: 5min, 15min, 30min, 45min, 60min, 90min, 120min, 180min;
5) Detection method: high performance liquid chromatography, detection wavelength: 254nm;
6) Preparation of test solution: 5ml of the solution was taken at each time point and filtered.

Preparation of reference sample solution: about 25 mg of the reference sample was accurately weighted and placed in a 100 mL volumetric flask. To the volumetric flask was added about 2 mL of DMSO. The reference sample was dissolved by ultrasound. Then the mixture was diluted to scale with the diluent of the corresponding medium, and shaken well.

### 2. Instrument model:

| **Name** | **Manufacturer/Model** | **Device number** |
|---|---|---|
| Intelligent dissolution apparatus | Swiss SOTAX AT Xtend | II050-808 |
| Intelligent dissolution apparatus | Swiss SOTAX AT Xtend | II050-809 |

### 3. Dissolution results

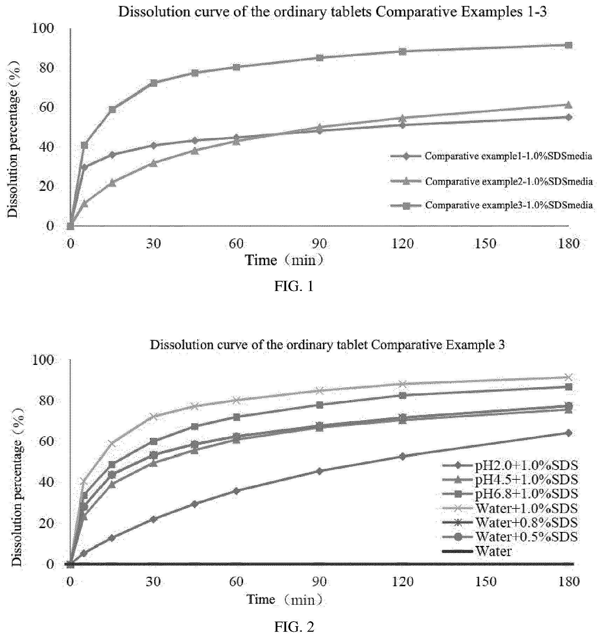

The dissolution results of Comparative Examples 1 to 3 in each medium are shown in Table 1 and FIG. 1 to FIG. 2:

**Table 1: Dissolution results of comparative examples 1 to 3**

| Time | Medium | 5min | 15min | 30min | 45min | 60min | 90min | 120min | 180min |
|---|---|---|---|---|---|---|---|---|---|
| Comparative example 1 | Water+1.0%SDS | 29.5 | 36.0 | 40.7 | 43.2 | 44.7 | 48.1 | 51.0 | 55.0 |
| Comparative | | 11.3 | 22.0 | 31.7 | 38.1 | 42.8 | 49.9 | 54.6 | 61.3 |
| example 2 | | | | | | | | | |
| Comparative example 3 | | 40.8 | 59 | 72.2 | 77.3 | 80.2 | 84.9 | 88.3 | 91.5 |
| Comparative example 3 | Ph6.8+ 1.0%SDS | 33.8 | 49 | 60.1 | 67.3 | 72.1 | 77.9 | 82.7 | 86.8 |
| Comparative example 3 | Ph4.5+ 1.0%SDS | 23.3 | 39.2 | 49.7 | 55.8 | 61 | 66.8 | 70.5 | 75.7 |
| Comparative example 3 | Ph2.0+ 1.0%SDS | 5.4 | 12.8 | 21.9 | 29.4 | 35.9 | 45.6 | 52.8 | 64.2 |
| Comparative example 3 | Water+0.8%SDS | 31.6 | 49.5 | 61.4 | 68.3 | 73.3 | 79.9 | 84.7 | 90.9 |
| Comparative example 3 | Water+0.5%SDS | 28.2 | 43.9 | 53.4 | 58.7 | 62.5 | 67.7 | 71.7 | 77.4 |
| Comparative example 3 | Water | ND | ND | ND | ND | ND | ND | ND | ND |

| | | | | | | | | | |
|---|---|---|---|---|---|---|---|---|---|
| Note: The dissolution results in the table are in percentage. "ND" means not detected. | | | | | | | | | |

It can be seen from Table 1 and FIGs. 1 to 2 that the ordinary API tablets prepared in Comparative Example 3 of the present disclosure were insoluble in purified water, and had significantly different dissolution rates in water and media with different pH values containing different concentrations of sodium dodecyl sulfate (SDS). As the proportion of SDS increased, the dissolution rate increased. When the pH was lower, drug aggregation was more serious and the dissolution rate was lower. Compared with Comparative Examples 1 to 3, the dissolution of the tablets prepared after micronizing the API was not significantly improved. After an appropriate amount of silicon dioxide was added and the formula was optimized (Comparative Example 3), the dissolution of the product was greatly improved, but the dissolution rate was still low in the medium with SDS concentration ≤0.5%.

### Test II

### Dissolution testing of nanocrystal suspensions in Examples 8 and 9

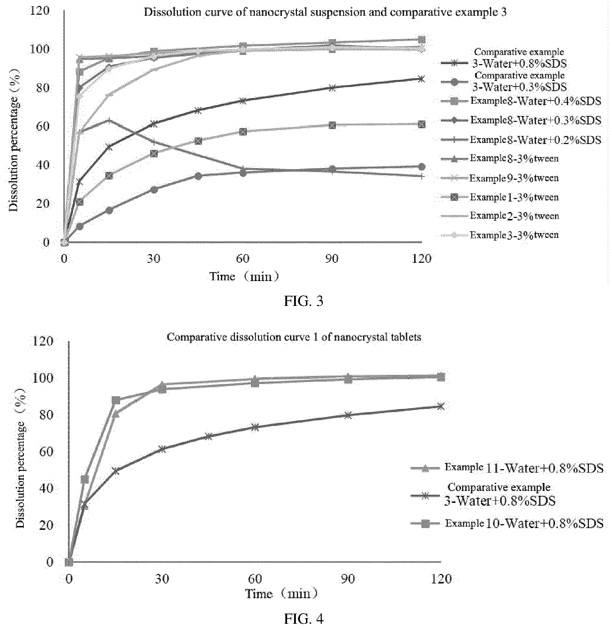

The dissolution results in each medium are shown in Table 2 and FIG. 3:

**Table 2: Dissolution results of nanocrystal suspensions in Examples 8 and 9**

| Time | Medium | 5min | 15min | 30min | 60min | 90min | 120min |
|---|---|---|---|---|---|---|---|
| Comparati | Water+0.8%SDS | 31.6 | 49.5 | 61.4 | 73.3 | 79.9 | 84.7 |
| ve example 3 | Water+0.3%SDS | 8.4 | 16.8 | 27.4 | 36.2 | 38.1 | 39.4 |
| Example 8 | Water+0.2%SDS | 57.0 | 63.1 | 52.0 | 38.2 | 36.7 | 34.3 |
| | Water+0.3%SDS | 80.0 | 90.8 | 95.5 | 99.6 | 101.8 | 100.1 |
| | Water+0.4%SDS | 88.4 | 95.4 | 98.9 | 101.7 | 103.3 | 105.1 |
| Example 8 | 3% Tween | 94.9 | 95.2 | 96.3 | 99.1 | 100.1 | 101.0 |
| Example 9 | | 95.9 | 96.3 | 97.9 | 99.5 | 101.0 | 100.5 |

| | | | | | | | |
|---|---|---|---|---|---|---|---|
| Note: The dissolution results in the table are in percentage. | | | | | | | |

As can be seen from Table 2 and FIG. 3, the dissolution rate of the nanocrystal suspension formulations prepared in Examples 8-9 of the present disclosure was significantly higher than that of ordinary tablets, and the dissolution rate reached 85% or more at 15 minutes in the medium with low SDS concentration (3%). But when the SDS concentration was further reduced to 2%, the sample precipitated in the later stage of dissolution. When using 3% Tween medium, the dissolution rates of the two nanocrystal suspensions in Example 8 and Example 9 were significantly improved, and the samples were stable without precipitation.

### Test III

### Dissolution test of nanocrystal tablets in Examples 10-15

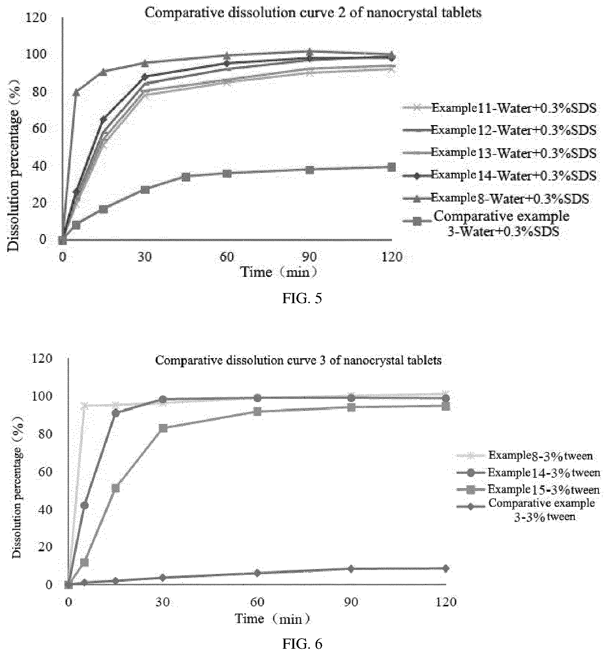

The dissolution results in each medium are shown in Table 3 and FIGs. 4 to 6:

**Table 3: Dissolution results of nanocrystal tablets in Examples 10-15**

| Time | Medium | 5min | 15min | 30min | 60min | 90min | 120min |
|---|---|---|---|---|---|---|---|
| Comparative example 3 | Water+0.8%SD S | 31.6 | 49.5 | 61.4 | 73.3 | 79.9 | 84.7 |
| | Water+0.3%SD S | 8.4 | 16.8 | 27.4 | 36.2 | 38.1 | 39.4 |
| | 3% Tween | 1.1 | 2.1 | 3.8 | 6.1 | 8.4 | 8.5 |
| Example 1 | 3% Tween | 21.2 | 34.6 | 46.2 | 52.7 | 57.4 | 60.8 |
| Example 2 | | 56.8 | 76.3 | 89.4 | 96.5 | 99.2 | 99.9 |
| Example 3 | | 75.6 | 89.9 | 96.3 | 99.1 | 99.8 | 100.8 |
| Example 8 | Water+0.3%SD S | 80.0 | 90.8 | 95.5 | 99.6 | 101.8 | 100.1 |
| Example 10 | Water+0.8%SD S | 44.9 | 88.1 | 94.0 | 97.5 | 99.4 | 100.7 |
| Example 11 | Water+0.8%SD S | 30.6 | 80.9 | 96.7 | 99.6 | 100.9 | 101.3 |
| | Water+0.3%SD S | 19.4 | 51.2 | 78.2 | 85.1 | 90 | 92.1 |
| Example 12 | Water+0.3%SD S | 22.8 | 58.0 | 84.2 | 92.1 | 97 | 99.2 |
| Example 13 | Water+0.3%SD S | 21.7 | 54.1 | 80.5 | 86.4 | 92.3 | 93.9 |
| Example 14 | Water+0.3%SD S | 26.0 | 65.2 | 88.0 | 95.3 | 98.1 | 98.2 |
| | 3% Tween | 42.2 | 91.2 | 98.4 | 99.1 | 99.0 | 98.8 |
| Example 15 | 3% Tween | 11.9 | 51.2 | 83.0 | 91.9 | 94.1 | 94.9 |

| | | | | | | | |
|---|---|---|---|---|---|---|---|
| Note: The dissolution results in the table are in percentage. | | | | | | | |

It can be seen from Table 3 and FIGs. 4 to 6 that the dissolution rate of the nanocrystal tablets prepared in Examples 10-15 of the present disclosure was significantly higher than that of ordinary tablets. Compared with the nanocrystal suspension, the dissolution rate in the early stage was slower, and the dissolution rate at 2 hours was similar.

### Test IV

### Dissolution test of nanocrystal capsules in Examples 24-25

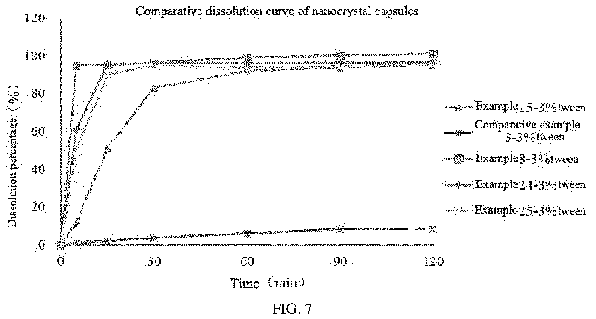

The dissolution results in each medium are shown in Table 4 and FIG. 7:

**Table 4: Dissolution results of nanocrystal capsules in Examples 24-25**

| Time | Medium | 5min | 15min | 30min | 60min | 90min | 120min |
|---|---|---|---|---|---|---|---|
| Comparati ve example 3 | 3% Tween | 1.1 | 2.1 | 3.8 | 6.1 | 8.4 | 8.5 |
| Example 8 | 3% Tween | 94.9 | 95.2 | 96.3 | 99.1 | 100.1 | 101.0 |
| Example 15 | 3% Tween | 11.9 | 51.2 | 83.0 | 91.9 | 94.1 | 94.9 |
| Example 24 | 3% Tween | 61.1 | 95.6 | 96.3 | 96.2 | 96.4 | 96.6 |
| Example 25 | 3% Tween | 50.7 | 90.1 | 94.8 | 93.8 | 94.5 | 95.5 |

It can be seen from Table 4 and FIG. 7 that the dissolution rate of the nanocrystal capsules prepared in Examples 24-25 of the present disclosure was significantly higher than that of ordinary tablets. Compared with nanocrystal tablets, the dissolution was accelerated at 1 hour and before, and the dissolution rate was similar after 1 hour. Compared with nanocrystal suspension, the dissolution was slower at 15 minutes and before, and the dissolution rate was similar after 15 minutes.

### Test V

### Dissolution test of nanocrystal enteric formulations in Examples 26-28

In this test, compared with other examples and comparative examples, the product specification was changed from 200 mg to 100 mg, and the corresponding dissolution medium volume was changed from 900 ml to 500 ml.

### The dissolution results in each medium are shown in Table 5:

**Table 5: Dissolution results of nanocrystal capsules in Examples 24-25**

| Time | Medium | 5min | 10min | 15min | 30min | 60min | 90min | 120min |
|---|---|---|---|---|---|---|---|---|
| Example 26 - Enteric tablets | 3% Tween + pH6.8 | 34.7 | 40.8 | 46.4 | 55.8 | 65.4 | 70.0 | 74.1 |
| | 1.0%SDS+pH6.8 | 38.0 | 67.3 | 72.0 | 83.9 | 89.7 | 94.7 | 96.1 |
| | 1.5%SDS+pH6.8 | 31.0 | 69.8 | 76.6 | 88.2 | 95.1 | 95.9 | 96.0 |
| Example 27 - Enteric tablets | 3% Tween + pH6.8 | 2.8 | 8.3 | 14.4 | 32.6 | 53.0 | 61.1 | 64.3 |
| | 1.5%SDS+pH6.8 | 1.9 | 7.8 | 15.8 | 32.0 | 67.5 | 89.3 | 94.4 |
| Example 28 - Enteric capsules | 3% Tween + pH6.8 | 5.7 | 13.8 | 22.9 | 38.0 | 64.2 | 72.1 | 76.0 |
| | 1.5%SDS+pH6.8 | 76.0 | 81.1 | 84.7 | 90.4 | 94.1 | 96.5 | 95.6 |

### Particle size stability test

In the process of preparing nanocrystal suspension, the inventor investigated the effects of different stabilizers on the particle size stability of the product; the specific test results are shown in Table 6 and Table 7.

**Table 6 Particle size distribution of nanocrystal suspensions in different stabilizers**

| **Accelerated testing** | **Zero hour** | | **40°C 5 days** | | **60°C 5 days** | |
|---|---|---|---|---|---|---|
| Sample | Particle size(nm) | PDI | Particle size(nm) | PDI | Particle size (nm) | PDI |
| Example 4, povidone K29/32 (10 mg/mL) | 300.3 | 0.176 | 267.2 | 0.199 | 275.7 | 0.195 |
| Example 5, povidone K29/32 (20 mg/mL) | 302.4 | 0.202 | 283.0 | 0.195 | 282.3 | 0.192 |
| Example 6, hypromellose (10 mg/mL) | 336.1 | 0.209 | 323.5 | 0.173 | 403.7 | 0.237 |
| Example 7, hypromellose (20 mg/mL) | 441.6 | 0.241 | 405.9 | 0.225 | 414.2 | 0.227 |

**Table 7: Average particle size results of suspensions before and after drying of nanocrystal powders with different stabilizers added after grinding**

| Example | Exam ple 16 | Exam ple 17 | Exam ple 18 | Exam ple 19 | Exam ple 20 | Exam ple 21 | Exam ple 22 | Exam ple 23 | -- | -- | -- |
|---|---|---|---|---|---|---|---|---|---|---|---|
| Stabilizer type | Polyso rbate 80 | Povid one K29/3 2 | Polyet hylene glycol 6000 | Polox amer 188 | Polyvi nyl alcoho 1 | Povid one K29/3 2/polo xamer 188 (4:1) | Povid one K29/3 2/polo xamer 188 (1:4) | Povid one K29/3 2/polo xamer 188 (1:1) | Polyet hylene glycol 15-hy droxys tearate | Polyo xyethy lene castor oil | Copov idone S630 |
| Average particle size (nm) of suspensio n before nanocryst al drying | 103 | 106 | 104 | 117 | 145 | 110 | 115 | 113 | 104 | 103 | 112 |
| After the nanocryst als were dried, the average particle size (nm) after the powder | 128 | 115 | 149 | 123 | 153 | 118 | 118 | 119 | 133 | 139 | 110 |
| was reconstitu ted with water | | | | | | | | | | | |
| After the nanocryst als were dried, the average particle size (nm) after the powder was reconstitu ted with pH 1.2 hydrochlo ric acid solution | 214 | 119 | 264 | 117 | 151 | 115 | 115 | 116 | 207 | 154 | 293 |
| After the nanocryst als were dried, the average particle size (nm) after the powder was reconstitu ted with pH 5.0 acetate buffer | 489 | 306 | 622 | 136 | 285 | 141 | 134 | 136 | 1979 | 3250 | - |

| | | | | | | | | | | | |
|---|---|---|---|---|---|---|---|---|---|---|---|
| Note: " -- " means not detected. | | | | | | | | | | | |

In the process of preparing nanocrystals, the inventor investigated the effects of grinding speed, grinding bead filling amount, sample amount and grinding time on API particle size.

**Table 8: Investigation results of grinding parameters and particle size**

| **No.** | | **I** | | **II** | | **III** | | **IV** | | **V** | | **VI** | |
|---|---|---|---|---|---|---|---|---|---|---|---|---|---|
| Grinding beads | | 0.2mm zirconia grinding beads | | 0.2mm zirconia grinding beads | | 0.2mm zirconia grinding beads | | 0.2mm zirconia grinding beads | | 0.2mm zirconia grinding beads | | 0.2mm zirconia grinding beads | |
| Grinding bead filling amount | | 70% (105mL) | | 70% (105mL) | | 80% (120mL) | | 90% (135mL) | | 90% (135mL) | | 70% (105mL) | |
| Grinding amount | | 1108 g (API: 26.0%) | | 967 g (API: 25.8%) | | 1097 g (API: 25.3%) | | 456 g (API: 27.0%) | | 221 g (API: 25.1%) | | 751g (API: 26.6%) | |
| Grinding parameters | | Grinding speed (rpm) | Particle size (nm) | Grinding speed (rpm) | Particle size (nm) | Grinding speed (rpm) | Particle size (nm) | Grinding speed (rpm) | Particle size(nm) | Grinding speed (rpm) | Particle size(nm) | Grinding speed (rpm) | Particle size(nm) |
| Grinding time | 3-5 min | 4000 | -- | 4000 | -- | 4000 | -- | 4000 | 2708 | 4000 | -- | 4000 | -- |
| | 1 h | 1500 | 458.9 | 1500 | 360.3 | 1500 | 369.1 | 3000 | 172.2 | 3000 | -- | 3000 | 190.5 |
| | 2 h | 1500 | 291.4 | 1500 | 348.6 | 1500 | 306.6 | 3000 | 131.2 | 3000 | 138.0 | 3000 | 160.4 |
| | 3 h | 1500 | 317.8 | 1500 | 354.3 | 1500 | 275.3 | 3000 | 112.1 | 3000 | 105.6 | 3000 | 136.8 |
| | 4 h | 1500 | 282.7 | 1500 | 280.8 | 1500 | 251.8 | 3000 | 100.1 | 3000 | 96.4 | 3000 | 124.2 |
| | 5 h | 1500 | 279.8 | -- | -- | -- | -- | -- | -- | -- | -- | 3000 | 116.6 |
| | 6 h | 1000 | 268.0 | -- | -- | -- | -- | -- | -- | -- | -- | 3000 | 108.8 |

| | | | | | | | | | | | | | |
|---|---|---|---|---|---|---|---|---|---|---|---|---|---|
| **[00302]** Note: " -- " means not detected. Initially pre-ground at 4000 rpm for 5 minutes to break the aggregate sample and disperse the drug quickly and evenly, thereby reducing the pressure in the grinding chamber and preventing clogging. | | | | | | | | | | | | | |

## Claims

1. A nanocrystal formulation, which comprises a ROCK2 inhibitor and a stabilizer, wherein the ROCK2 inhibitor is a compound of formula (I), 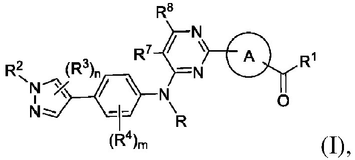 wherein
Ring A is 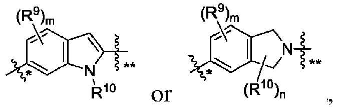 wherein the above group is connected to the pyrimidine ring through one of the two positions marked by* or **, and the other position is connected to the carbonyl group;
R⁹ and R¹⁰ at each occurrence are each independently selected from H, halogen, C₁₋₆ alkyl, C₂₋₆ alkenyl, C₃₋₁₀ carbocyclyl, 3-10 membered heterocyclyl, C₆₋₁₀ aryl, 5-14 membered heteroaryl, C₆₋₁₂ aralkyl, -C(=O)R⁵ and -C₁₋₆ alkylene-O(P=O)(OH)₂;
m at each occurrence is independently an integer of 0, 1, 2, or 3; and
n at each occurrence is independently an integer of 0, 1 or 2;
alternatively, ring A is 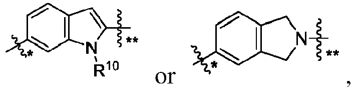 wherein the above group is connected to the pyrimidine ring through the position marked by *, and is connected to the carbonyl group through the position marked by **, wherein R¹⁰ is selected from H and C₁₋₆ alkyl, alternatively H or methyl;
R is selected from H and C₁₋₆ alkyl;
R¹ is 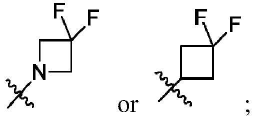
R² is selected from H and C₁₋₆ alkyl;
R³, R⁴, R⁷ and R⁸ at each occurrence are each independently selected from H, halogen, -NR⁵R⁶, -OH, C₁₋₆ alkyl and -OR⁵;
each of the above-mentioned alkylene, alkyl, alkenyl, carbocyclyl, heterocyclyl, aryl, heteroaryl and aralkyl groups at each occurrence is optionally substituted with one or more substituents independently selected from halogen, C₁₋₆ alkyl and -OR⁵;
R⁵ and R⁶ at each occurrence are each independently selected from H, C₁₋₆ alkyl, C₃₋₁₀ carbocyclyl, 3-10 membered heterocyclyl, C₆₋₁₀ aryl, 5-14 membered heteroaryl and C₆₋₁₂ aralkyl;
or pharmaceutically acceptable salts, esters, stereoisomers, polymorphs, solvates, N-oxides, isotopically labeled derivatives, metabolites or prodrugs thereof.

2. The nanocrystal formulation according to claim 1, wherein the ROCK2 inhibitor is a compound of formula (II) or pharmaceutically acceptable salts, esters, stereoisomers, polymorphs, solvates, N-oxides, isotopically labeled derivatives, metabolites or prodrugs thereof, 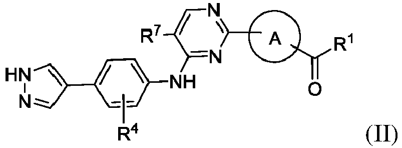 wherein each group is as defined in claim 1.

3. The nanocrystal formulation according to any one of claims 1-2, wherein the ROCK2 inhibitor is a compound of formula (III) or pharmaceutically acceptable salts, esters, stereoisomers, polymorphs, solvates, N-oxides, isotopically labeled derivatives, metabolites or prodrugs thereof, 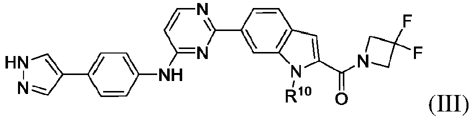 wherein R¹⁰ is H or methyl, alternatively methyl.

4. The nanocrystal formulation according to any one of claims 1-3, wherein the ROCK2 inhibitor is a compound of formula (IV) or pharmaceutically acceptable salts, esters, stereoisomers, polymorphs, solvates, N-oxides, isotopically labeled derivatives, metabolites or prodrugs thereof,

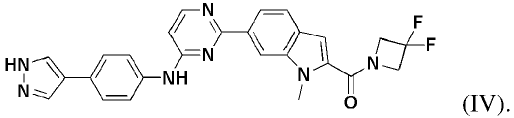

5. The nanocrystal formulation according to any one of claims 1 to 4, wherein the stabilizer is selected from one or more of polysorbate, povidone, polyoxyethylene fatty acid ester, polyethylene glycol, polyvinyl alcohol, hydroxypropyl cellulose, hydroxypropyl methylcellulose, polyvinylpyrrolidone, poloxamer, sodium lauryl sulfate, sodium docusate, polyethylene glycol 15-hydroxystearate, polyoxyethylene castor oil, copovidone, lactose, and mannitol; alternatively, the stabilizer is selected from one or more of polysorbate, povidone, hydroxypropyl methylcellulose, polyethylene glycol 6000, polyvinyl alcohol, polyoxyethylene castor oil, poloxamer and sodium lauryl sulfate, lactose and mannitol.

6. The nanocrystal formulation according to any one of claims 1-5, wherein the particle size D₉₀ of the nanocrystal formulation is 50-1500nm, alternatively 50-1000nm, alternatively 50-500nm, alternatively 80-300nm, yet alternatively 50nm, 100nm, 150nm, 200nm, 250nm, 300nm, 400nm, 500nm, 600nm, 700nm, 800nm, 900nm or 1000nm.

7. The nanocrystal formulation according to any one of claims 1-6, wherein the weight percentage of the ROCK2 inhibitor is 1%-55%, alternatively 4%-50%, alternatively 1%-10%, alternatively 10%-40%, alternatively 10%-35%, alternatively 20%-30%, alternatively 30%-40%, yet alternatively 4%, 4.5%, 5%, 10%, 15%, 20%, 25%, 30%, 35% or 40%.

8. The nanocrystal formulation according to any one of claims 1-7, wherein the weight percentage of the stabilizer is 0.1%-55%, alternatively 0.1%-30%, alternatively 0.5%-1%, 1% -10%, 10%-20% or 20%-30%, yet alternatively 1%, 2%, 5%, 10%, 15%, 20% or 30%.

9. The nanocrystal formulation according to any one of claims 1-8, wherein the weight ratio of the ROCK2 inhibitor to the stabilizer is 1:10 to 10:1, alternatively 1:9 to 9:1, alternatively 1:8 to 8:1, alternatively 1:7 to 7:1, alternatively 1:6 to 6:1, alternatively 1:5 to 5:1, alternatively 1:4 to 4:1, alternatively 1:3 to 3:1, alternatively 1:2 to 2:1, alternatively 1:1; alternatively, the weight ratio of the ROCK2 inhibitor to the stabilizer is 4:1 to 1:1 or 1:1 to 1:2, yet alternatively 5:4, 5:3, 4:1, 3:1, 2:1, 1:1, 1:2 or 1:3.

10. The nanocrystal formulation according to any one of claims 1 to 9, wherein the nanocrystal formulation comprises an excipient.

11. The nanocrystal formulation according to claim 10, wherein the excipient is selected from one or more of fillers; wetting agents; sweeteners or flavoring agents; surfactants; binders; disintegrants; lubricants; glidants or anti-adhesion agents; release modifiers; coating agents; emulsifiers; solubilizers; and fragrances.

12. The nanocrystal formulation according to claim 10 or 11, wherein the excipient comprises a filler selected from one or more of microcrystalline cellulose, mannitol, lactose, starch, pregelatinized starch, dextrin, calcium phosphate dihydrate and anhydrous calcium hydrogen phosphate.

13. The nanocrystal formulation according to any one of claims 10-12, wherein the excipient includes a filler, and the amount of the filler is 1% to 80%, alternatively 20% to 70%, yet alternatively 30% to 60% or 50% to 70%.

14. The nanocrystal formulation according to any one of claims 11-13, wherein the excipient comprises a lubricant selected from one or more of magnesium stearate, talc powder, micronized silica gel, sodium stearyl fumarate, glyceryl behenate and polyethylene glycol.

15. The nanocrystal formulation according to any one of claims 11 to 14, wherein the excipient comprises a lubricant, and the amount of the lubricant is 0.1% to 5%, alternatively 0.1% to 1.5%, yet alternatively 0.5% to 1%.

16. The nanocrystal formulation according to any one of claims 11-15, wherein the excipient comprises a disintegrant selected from one or two of croscarmellose sodium and crospovidone;
alternatively, the amount of the disintegrant is 0 to 20%, alternatively 0 to 10%, yet alternatively 2 to 10%.

17. The nanocrystal formulation according to any one of claims 11-16, wherein the excipient comprises a glidant selected from silicon dioxide;
alternatively, the amount of the glidant is 0 to 20%, alternatively 0 to 15%, yet alternatively 2 to 12%.

18. The nanocrystal formulation according to any one of claims 1 to 17, wherein the nanocrystal formulation further comprises a solvent;
alternatively, the solvent is water, alternatively purified water;
alternatively, the amount of the solvent is 0 to 99%, alternatively 80 to 99%, yet alternatively 85% to 95%.

19. The nanocrystal formulation according to any one of claims 1 to 18, wherein the nanocrystal formulation also comprises a bacteriostatic agent;
alternatively, the bacteriostatic agent is selected from one or two of methylparaben and propylparaben;
alternatively, the amount of the bacteriostatic agent is 0 to 5%, alternatively 0 to 1%, yet alternatively 0.01% to 0.5%.

20. The nanocrystal formulation according to any one of claims 1 to 19, wherein the nanocrystal formulation is selected from suspensions, tablets, capsules, granules, powders, lozenges and pills; alternatively, suspensions, tablets or capsules.

21. The nanocrystal formulation according to any one of claims 1 to 20, wherein the nanocrystal formulation is a suspension, comprising:
- 1-10% of ROCK2 inhibitor, alternatively 1%, 2%, 3%, 4%, 4.5%, 5%, 6%, 7%, 8%, 9% or 10% of ROCK2 inhibitor, yet alternatively 4%, 4.5%, or 5% of ROCK2 inhibitor; and
- 1-10% of stabilizer, alternatively 1%, 1.5%, 2%, 2.5%, 5%, 9%, 9.5% or 10% of stabilizer.

22. The nanocrystal formulation according to claim 21, wherein the nanocrystal is a suspension, comprising 11.13g of ROCK2 inhibitor, 0.77g of polysorbate 80, 5.00g of povidone K29/32 and 233.10g of purified water, alternatively, the particle size of the ROCK2 inhibitor is 50-1000nm, alternatively 50-500nm, yet alternatively 50-300nm.

23. The nanocrystal formulation according to claim 21, wherein the nanocrystal is a suspension, comprising 11.13g of ROCK2 inhibitor, 0.77g of polysorbate 80, 2.50g of povidone K29/32 and 235.60g of purified water, alternatively, the particle size of the ROCK2 inhibitor is 50-1000nm, alternatively 50-500nm, yet alternatively 50-300nm.

24. The nanocrystal formulation according to claim 21, wherein the nanocrystal is a suspension, comprising 11.13g of ROCK2 inhibitor, 0.77g of polysorbate 80, 2.50g of hypromellose and 235.60g of purified water, alternatively, the particle size of the ROCK2 inhibitor is 50-1000 nm, alternatively 50-500 nm, yet alternatively 50-300 nm.

25. The nanocrystal formulation according to claim 21, wherein the nanocrystal is a suspension, comprising 11.13g of ROCK2 inhibitor, 0.77g of polysorbate 80, 5.00g of hypromellose and 233.10g of purified water, alternatively, the particle size of the ROCK2 inhibitor is 50-1000 nm, alternatively 50-500 nm, yet alternatively 50-300 nm.

26. The nanocrystal formulation according to claim 21, wherein the nanocrystal is a suspension, comprising 250.08g of ROCK2 inhibitor, 17.40g of polysorbate 80, 111.60g of povidone K29/32, 10.00g of methylparaben, 1.10g of propylparaben and 5189.82g of purified water, alternatively, the particle size of the ROCK2 inhibitor is 50-1000nm, alternatively 50-500nm, yet alternatively 50-300nm.

27. The nanocrystal formulation according to claim 21, wherein the nanocrystal is a suspension, comprising 55.19g of ROCK2 inhibitor, 18.61g of polysorbate 80, 99.25g of polyoxyethylene castor oil, 2.23g of methylparaben, 0.25g of propylparaben and 1065.09g of purified water, alternatively, the particle size of the ROCK2 inhibitor is 50-1000nm, alternatively 50-500nm, yet alternatively 50-300nm, still alternatively 50-150nm.

28. The nanocrystal formulation according to any one of claims 1-20, wherein the nanocrystal formulation is a tablet, comprising:
- 10-30% of ROCK2 inhibitor, alternatively 20-30% of ROCK2 inhibitor, alternatively 20%, 22%, 25%, 28% or 30% of ROCK2 inhibitor;
- 1-20% of stabilizer, alternatively 5-20% of stabilizer, yet alternatively 5%, 8%, 10%, 13%, 15%, 18% or 20% of stabilizer.

29. The nanocrystal formulation according to claim 28, wherein the nanocrystal is a tablet, comprising 11.70g of ROCK2 inhibitor, 3.15g of polysorbate 80, 5.26g of lactose, 1.05g of polyethylene glycol 6000, 16.03g of mannitol, 4.94g of silicon dioxide, 2.47g of sodium lauryl sulfate, 2.96g of microcrystalline cellsulose, 2.96g of croscarmellose sodium and 0.32g of magnesium stearate, alternatively, the particle size of the ROCK2 inhibitor is 50-1000nm, alternatively 50-500nm, yet alternatively 50-300nm, still alternatively 50-150nm.

30. The nanocrystal formulation according to claim 28, wherein the nanocrystal is a tablet, comprising 32.71g of ROCK2 inhibitor, 14.81g of polysorbate 80, 14.71g of lactose, 2.94g of polyethylene glycol 6000, 43.63g of mannitol, 16.00g of silicon dioxide, 8.00g of sodium lauryl sulfate, 12.80g of microcrystalline cellulose, 12.80g of croscarmellose sodium and 1.60g of sodium stearyl fumarate, alternatively, the particle size of the ROCK2 inhibitor is 50-1000nm, alternatively 50-500nm, yet alternatively 50-300nm, still alternatively 50-150nm.

31. The nanocrystal formulation according to claim 28, wherein the nanocrystal is a tablet, comprising 3.34g of ROCK2 inhibitor, 0.89g of polysorbate 80, 1.80g of lactose, 0.30g of polyethylene glycol 6000, 0.75g of silicon dioxide, 0.75g of sodium lauryl sulfate, 5.82g of microcrystalline cellulose, 1.20g of croscarmellose sodium and 0.15g of sodium stearyl fumarate, alternatively, the particle size of the ROCK2 inhibitor is 50-1000nm, alternatively 50-500nm, yet alternatively 50-300nm, still alternatively 50-150nm.

32. The nanocrystal formulation according to claim 28, wherein the nanocrystal is a tablet, comprising 3.34g of ROCK2 inhibitor, 0.89g of polysorbate 80, 1.80g of lactose, 0.30g of polyethylene glycol 6000, 0.75g of silicon dioxide, 0.45g of sodium lauryl sulfate, 4.50g of microcrystalline cellulose, 1.62g of pregelatinized starch, 1.20g of croscarmellose sodium and 0.15g of sodium stearyl fumarate, alternatively, the particle size of the ROCK2 inhibitor is 50-1000nm, alternatively 50-500nm, yet alternatively 50-300nm, still alternatively 50-150nm.

33. The nanocrystal formulation according to claim 28, wherein the nanocrystal is a tablet, comprising 11.14g of ROCK2 inhibitor, 2.97g of polysorbate 80, 6.01g of lactose, 1.00g of polyethylene glycol 6000, 1.00g of silicon dioxide, 27.38g of spray-dried mannitol and 0.50g of sodium stearyl fumarate, alternatively, the particle size of the ROCK2 inhibitor is 50-1000nm, alternatively 50-500nm, yet alternatively 50-300nm, still alternatively 50-150nm.

34. The nanocrystal formulation according to claim 28, wherein the nanocrystal is a tablet, comprising 111.20g of ROCK2 inhibitor, 30.04g of polysorbate 80, 59.99g of lactose, 10.00g of polyethylene glycol 6000, 10.00g of silicon dioxide, 273.78g of spray-dried mannitol and 5.00g of sodium stearyl fumarate, alternatively, the particle size of the ROCK2 inhibitor is 50-1000nm, alternatively 50-500nm, yet alternatively 50-300nm, still alternatively 50-150nm.

35. The nanocrystal formulation according to any one of claims 1-20, wherein the nanocrystal formulation is a tablet, comprising:
- 10-30% of ROCK2 inhibitor, alternatively 20-30% of ROCK2 inhibitor, yet alternatively 20%, 22%, 25%, 28% or 30% of ROCK2 inhibitor;
- 10-30% of stabilizer, alternatively 20-30% of stabilizer, yet alternatively 20%, 22%, 25%, 28% or 30% of stabilizer.

36. The nanocrystal formulation according to claim 35, wherein the nanocrystal is a tablet, comprising 22.22g of ROCK2 inhibitor, 20.00g of polysorbate 80, 54.80g of mannitol, 2.00g of silicon dioxide and 1.00g sodium stearyl fumarate, alternatively, the particle size of the ROCK2 inhibitor is 50-1000nm, alternatively 50-500nm, yet alternatively 50-300nm, still alternatively 50-150nm.

37. The nanocrystal formulation according to claim 35, wherein the nanocrystal is a tablet, comprising 22.20g of ROCK2 inhibitor, 10.00g of polysorbate 80, 20.00g of povidone K29/32, 44.80g of mannitol, 2.00g of silicon dioxide and 1.00g of sodium stearyl fumarate, alternatively, the particle size of the ROCK2 inhibitor is 50-1000nm, alternatively 50-500nm, yet alternatively 50-300nm, still alternatively 50-150nm.

38. The nanocrystal formulation according to claim 35, wherein the nanocrystal is a tablet, comprising 22.20g of ROCK2 inhibitor, 10.00g of polysorbate 80, 20.00g of polyethylene glycol 6000, 44.80g of mannitol, 2.00 g of silicon dioxide and 1.00 g of sodium stearyl fumarate, alternatively, the particle size of the ROCK2 inhibitor is 50-1000 nm, alternatively 50-500 nm, yet alternatively 50-300 nm, still alternatively 50-150 nm.

39. The nanocrystal formulation according to claim 35, wherein the nanocrystal is a tablet, comprising 22.20g of ROCK2 inhibitor, 10.00g of polysorbate 80, 20.00g of poloxamer 188, 44.80g of mannitol, 2.00 g of silicon dioxide and 1.00 g of sodium stearyl fumarate, alternatively, the particle size of the ROCK2 inhibitor is 50-1000 nm, alternatively 50-500 nm, yet alternatively 50-300 nm, still alternatively 50-150 nm.

40. The nanocrystal formulation according to claim 35, wherein the nanocrystal is a tablet, comprising 22.20g of ROCK2 inhibitor, 10.00g of polysorbate 80, 20.00g of polyvinyl alcohol, 44.80g of mannitol, 2.00g of silicon dioxide and 1.00g of sodium stearyl fumarate, alternatively, the particle size of the ROCK2 inhibitor is 50-1000nm, alternatively 50-500nm, yet alternatively 50-300nm, still alternatively 50-150nm.

41. The nanocrystal formulation according to claim 35, wherein the nanocrystal is a tablet, comprising 22.20g of ROCK2 inhibitor, 10.00g of polysorbate 80, 16.00g of povidone K29/32, 4.00g of poloxamer 188, 44.80g of mannitol, 2.00g of silicon dioxide and 1.00g of sodium stearyl fumarate, alternatively, the particle size of the ROCK2 inhibitor is 50-1000nm, alternatively 50-500nm, yet alternatively 50-300nm, still alternatively 50-150nm.

42. The nanocrystal formulation according to claim 35, wherein the nanocrystal is a tablet, comprising 22.20g of ROCK2 inhibitor, 10.00g of polysorbate 80, 4.00g of povidone K29/32, 16.00g of poloxamer 188, 44.80g of mannitol, 2.00g of silicon dioxide and 1.00g of sodium stearyl fumarate, alternatively, the particle size of the ROCK2 inhibitor is 50-1000nm, alternatively 50-500nm, yet alternatively 50-300nm, still alternatively 50-150nm.

43. The nanocrystal formulation according to claim 35, wherein the nanocrystal is a tablet, comprising 22.20g of ROCK2 inhibitor, 10.00g of polysorbate 80, 10.00g of povidone K29/32, 10.00g of poloxamer 188, 44.80g of mannitol, 2.00g of silicon dioxide and 1.00g of sodium stearyl fumarate, alternatively, the particle size of the ROCK2 inhibitor is 50-1000nm, alternatively 50-500nm, yet alternatively 50-300nm, still alternatively 50-150nm.

44. The nanocrystal formulation according to any one of claims 1 to 20, wherein the nanocrystal formulation is a capsule, comprising:
- 10-50% of ROCK2 inhibitor, alternatively 20-40% of ROCK2 inhibitor, yet alternatively 20%, 25%, 30%, 35% or 40% of ROCK2 inhibitor;
- 10-40% of stabilizer, alternatively 20-30% of stabilizer, yet alternatively 20%, 22%, 25%, 28% or 30% of stabilizer.

45. The nanocrystal formulation according to claim 44, wherein the nanocrystal is a capsule, comprising 22.20g of ROCK2 inhibitor, 6.00g of polysorbate 80, 8.00g of povidone K29/32, 4.00g of poloxamer and 20.00g of mannitol, alternatively, the particle size of the ROCK2 inhibitor is 50-1000nm, alternatively 50-500nm, yet alternatively 50-300nm, still alternatively 50-150nm.

46. The nanocrystal formulation according to claim 44, wherein the nanocrystal is a capsule, comprising 4.45g of ROCK2 inhibitor, 1.20g of polysorbate 80, 1.20g of povidone K32/29, 0.80g of poloxamer 188 and 6.80g of mannitol, alternatively, the particle size of the ROCK2 inhibitor is 50-1000nm, alternatively 50-500nm, yet alternatively 50-300nm, still alternatively 50-150nm.

47. The nanocrystal formulation according to any one of claims 1 to 19, wherein the nanocrystal formulation is a nanocrystal enteric formulation selected from enteric-coated tablets and enteric capsules.

48. The nanocrystal formulation according to claim 47, wherein the nanocrystal formulation is a nanocrystal enteric-coated tablet, wherein the enteric coating material is selected from one or more of shellac, polyvinyl alcohol acetate phthalate (PVAP), methacrylic acid copolymer, cellulose and its derivatives (cellulose acetate phthalate (CAP), cellulose acetate trimellitate (CAT), hydroxypropyl cellulose phthalate (HPMCP)), and acrylic resins (EuS100, EuL100).

49. The nanocrystal formulation according to claim 47, wherein the nanocrystal formulation is a nanocrystal enteric capsule, wherein the enteric capsule is selected from gelatin enteric capsules or hypromellose enteric capsules, and the capsule material composition is selected from one or more of shellac, polyvinyl alcohol acetate phthalate (PVAP), methacrylic acid copolymer, cellulose and its derivatives (cellulose acetate phthalate (CAP), cellulose acetate trimellitate (CAT), hydroxypropyl cellulose phthalate (HPMCP)) and acrylic resins (EuS100, EuL100).

50. The nanocrystal formulation according to any one of claims 1-19 and 47-48, wherein the nanocrystal formulation is a nanocrystal enteric-coated tablet, comprising:
- 1-20% of ROCK2 inhibitor, alternatively 5-15% of ROCK2 inhibitor, yet alternatively 5%, 6%, 7%, 8%, 9%, 10%, 11%, 12%, 13%, 14% or 15% of ROCK2 inhibitor; and
- 1-90% of stabilizer, alternatively 10-40% of stabilizer, yet alternatively 15-25% of stabilizer, for example, 15%, 16%, 17%, 18%, 19%, 20%, 21%, 22%, 23%, 24% or 25% of stabilizer.

51. The nanocrystal formulation according to claim 50, wherein the nanocrystal formulation is a nanocrystal enteric-coated tablet, comprising 5.56 g of ROCK2 inhibitor, 1.50 g of polysorbate 80, 1.00 g of poloxamer, 1.50 g of povidone K29/32, 5.00 g of mannitol, 1.00 g of silicon dioxide, 4.00 g of crospovidone, 29.94 g of microcrystalline cellulose, 0.50 g of magnesium stearate and 5g of film coating premix (enteric type), alternatively, the particle size of the ROCK2 inhibitor is 50-1000nm, alternatively 50-500nm, yet alternatively 50-300nm, still alternatively 50-150nm.

52. The nanocrystal formulation according to claim 50, wherein the nanocrystal formulation is a nanocrystal enteric-coated tablet, comprising 5.56 g of ROCK2 inhibitor, 1.50 g of polysorbate 80, 1.00 g of poloxamer, 1.50 g of povidone K29/32, 38.94 g of mannitol, 1.00 g of silicon dioxide, 0.50 g of magnesium stearate and 5g of film coating premix (enteric type), alternatively, the particle size of the ROCK2 inhibitor is 50-1000nm, alternatively 50-500nm, yet alternatively 50-300nm, still alternatively 50-150nm.

53. The nanocrystal formulation according to any one of claims 1-19, 47 and 49, wherein the nanocrystal formulation is a nanocrystal enteric capsule, comprising:
- 5-30% of ROCK2 inhibitor, alternatively 10-20% of ROCK2 inhibitor, yet alternatively 10%, 11%, 12%, 13%, 14%, 15%, 16%, 17%, 18%, 19% or 20% of ROCK2 inhibitor; and
- 10-50% of stabilizer, alternatively 20-40% of stabilizer, yet alternatively 30-40% of stabilizer, for example, 30%, 31%, 32%, 33%, 34%, 35%, 36%, 37%, 38%, 39% or 40% of stabilizer.

54. The nanocrystal formulation according to claim 53, wherein the nanocrystal formulation is a nanocrystal enteric capsule, comprising 11.1 g of ROCK2 inhibitor, 3.0 g of polysorbate 80, 2.0 g of poloxamer, 3.0 g of povidone K29/32, 20.0 g of mannitol, 2.00 g of silicon dioxide, 8.0 g of crospovidone, 29.9 g of microcrystalline cellulose, 1.0 g of magnesium stearate and enteric capsules, alternatively the particle size of the ROCK2 inhibitor is 50-1000nm, alternatively 50-500nm, yet alternatively 50-300nm, still alternatively 50-150nm.

55. A preparation method of the nanocrystal formulation according to claims 1-54, which comprises grinding the ROCK2 inhibitor and the stabilizer.

56. The preparation method according to claim 55, wherein the weight ratio of the ROCK2 inhibitor to the stabilizer during grinding is 1:15 to 15:1, 1:14 to 14:1, 1:13 to 13:1, 1:12 to 12:1, 1:11 to 11:1, 1:10 to 10:1, 1:9 to 9:1, 1:8 to 8:1, 1:7 to 7:1, 1:6 to 6:1, 1:5 to 5:1, 1:4 to 4:1, 1:3 to 3:1, 1:2 to 2:1, 1:1; alternatively, the weight ratio of the ROCK2 inhibitor to the stabilizer during grinding is 15:1 to 2:1, yet alternatively 15:1, 10:1, 10:3, 5:1, 4:1, 3:1 or 2:1.

57. The preparation method according to any one of claims 55-56, wherein the grinding medium is selected from porcelain balls, glass balls, zirconia beads, steel balls or ice beads; alternatively, the grinding medium is zirconia beads.

58. The preparation method according to any one of claims 55-57, wherein the particle size of the grinding medium ranges from 0.1-1mm, alternatively 0.1-0.5mm, yet alternatively 0.2mm.

59. The preparation method according to any one of claims 55-58, wherein the grinding time is 0.1-6h, alternatively 0.5-6h, alternatively 4-6h, yet alternatively 10min, 20min, 30min, 40min, 1h, 1.5 h, 2h, 2.5h, 3h, 3.5h, 4h, 4.5h, 5h, 5.5h or 6h.

60. The preparation method according to any one of claims 55-59, wherein the grinding speed is 1000∼6000rpm, alternatively 1500rpm∼4500rpm, yet alternatively 1500rpm, 2000rpm, 2500rpm, 3000rpm, 3500rpm, 4000rpm, 4500rpm, 5000rpm, 5500rpm or 6000rpm.

61. The preparation method according to any one of claims 55-60, wherein the filling amount of the grinding beads is 50% to 95%, alternatively 70% to 90%, yet alternatively 70%, 80% or 90%.

62. The preparation method according to any one of claims 55-61, further including a pre-grinding step before grinding the ROCK2 inhibitor and the stabilizer.

63. The preparation method according to claim 62, wherein the pre-grinding speed is 3000~6000rpm, alternatively 3000rpm, 3500rpm, 4000rpm, 4500rpm, 5000rpm, 5500rpm or 6000rpm, yet alternatively 4000rpm; the pre-grinding time is 1-30min, alternatively 2-20min, alternatively 3min, 4min, 5min, 6min, 8min, 10min, 12min, 15min, 18min or 20min, yet alternatively 5min.

64. The preparation method according to any one of claims 55-63, wherein a stabilizer and/or an excipient are optionally added after grinding.

65. The preparation method according to claim 64, wherein the stabilizer is selected from one or more of polysorbate, povidone, polyoxyethylene fatty acid ester, polyethylene glycol, polyvinyl alcohol, hydroxypropyl cellulose, hydroxypropyl methylcellulose, polyvinylpyrrolidone, poloxamer, sodium lauryl sulfate, sodium docusate, polyethylene glycol 15-hydroxystearate, polyoxyethylene castor oil, copovidone, lactose, and mannitol; alternatively, the stabilizer is selected from povidone K29/32, poloxamer 188, polyvinyl alcohol, lactose, and mannitol; alternatively, the stabilizer is a mixture of povidone K29/32 and poloxamer 188 with a mixing ratio of 1:10 to 10:1, alternatively 1:9 to 9:1, alternatively 1:8 to 8:1, alternatively 1:7 to 7:1, alternatively 1:6 to 6:1, alternatively 1:5 to 5:1, alternatively 1:4 to 4:1, alternatively 1:3 to 3:1, alternatively 1:2 to 2:1, alternatively 1:1; yet alternatively, 1:4, 4:1 or 1:1.

66. A method for preventing, alleviating and/or treating idiopathic pulmonary fibrosis, fatty liver disease and/or steatohepatitis, graft-versus-host disease after hematopoietic stem cell transplantion or viral infection, comprising administering to a subject a therapeutically effective amount of the nanocrystal formulation according to any one of claims 1-54 or the nanocrystal formulation prepared by the method according to any one of claims 55-65; alternatively, the method is a method for preventing, alleviating and/or treating fatty liver disease and/or steatohepatitis; alternatively, the fatty liver disease is alcoholic fatty liver disease (ALFD) or non-alcoholic fatty liver disease (NALFD), the steatohepatitis is alcoholic steatohepatitis (ASH) or non-alcoholic steatohepatitis (NASH), the hematopoietic stem cell transplantion is an allogeneic hematopoietic stem cell transplantion, the graft-versus-host disease is acute graft-versus-host disease or chronic graft-versus-host disease, and the viral infection is a coronavirus infection; alternatively, the coronavirus is selected from SARA-CoV, SARA-CoV-2, MERS-CoV, HCoV-229E, HCoV-NL63, HCoV-OC43 and HCoV-HKU1; alternatively, the disease caused by the coronavirus is Middle East Respiratory Syndrome, Severe Acute Respiratory Syndrome or COVID-19; alternatively, the coronavirus is Severe Acute Respiratory Syndrome coronavirus 2, and the disease caused by the coronavirus is COVID-19.

67. A nanocrystal formulation according to any one of claims 1-54 or a nanocrystal formulation prepared by the method according to any one of claims 55-65, for use in preventing, alleviating and/or treating idiopathic pulmonary fibrosis, fatty liver disease and /or steatohepatitis, graft-versus-host disease after hematopoietic stem cell transplantion or viral infection; alternatively, the nanocrystal formulation is used for preventing, alleviating and/or treating fatty liver disease and/or steatohepatitis; alternatively, the fatty liver disease is alcoholic fatty liver disease (ALFD) or non-alcoholic fatty liver disease (NALFD), the steatohepatitis is alcoholic steatohepatitis (ASH) or non-alcoholic steatohepatitis (NASH), the hematopoietic stem cell transplantion is an allogeneic hematopoietic stem cell transplantion, the graft-versus-host disease is acute graft-versus-host disease or chronic graft-versus-host disease, and the viral infection is a coronavirus infection; alternatively, the coronavirus is selected from SARA-CoV, SARA-CoV-2, MERS-CoV, HCoV-229E, HCoV-NL63, HCoV-OC43 and HCoV-HKU1; alternatively, the disease caused by the coronavirus is Middle East Respiratory Syndrome, Severe Acute Respiratory Syndrome or COVID-19; alternatively, the coronavirus is Severe Acute Respiratory Syndrome coronavirus 2, and the disease caused by the coronavirus is COVID-19.

68. Use of the nanocrystal formulation according to any one of claims 1-54 or the nanocrystal formulation prepared by the method according to any one of claims 55-65 in the manufacture of a medicament for preventing, alleviating and/or treating idiopathic pulmonary fibrosis, fatty liver disease and/or steatohepatitis, graft-versus-host disease after hematopoietic stem cell transplantion or viral infection; alternatively, the use is a use in the manufacture of a medicament for preventing, alleviating and/or treating fatty liver disease and/or steatohepatitis; alternatively, the fatty liver disease is alcoholic fatty liver disease (ALFD) or non-alcoholic fatty liver disease (NALFD), the steatohepatitis is alcoholic steatohepatitis (ASH) or non-alcoholic steatohepatitis (NASH), the hematopoietic stem cell transplantion is an allogeneic hematopoietic stem cell transplantion, the graft-versus-host disease is acute graft-versus-host disease or chronic graft-versus-host disease, and the viral infection is a coronavirus infection; alternatively, the coronavirus is selected from SARA-CoV, SARA-CoV-2, MERS-CoV, HCoV-229E, HCoV-NL63, HCoV-OC43 and HCoV-HKU1; alternatively, the disease caused by the coronavirus is Middle East Respiratory Syndrome, Severe Acute Respiratory Syndrome or COVID-19; alternatively, the coronavirus is Severe Acute Respiratory Syndrome coronavirus 2, and the disease caused by the coronavirus is COVID-19.
